(19) Europäisches Patentamt
European Patent Office
Office européen des brevets

(11) Numéro de publication: **0 429 372 A1**

(12) **DEMANDE DE BREVET EUROPEEN**

(21) Numéro de dépôt: **90420452.6**

(22) Date de dépôt: **18.10.90**

(51) Int. Cl.[5]: **C07D 401/04**, C07D 471/04, C07D 401/14, C07D 409/14, //(C07D471/04,249:00,221:00), (C07D471/04,235:00,221:00)

(30) Priorité: **20.10.89 FR 8914091**

(43) Date de publication de la demande: **29.05.91 Bulletin 91/22**

(84) Etats contractants désignés: **AT BE CH DE DK ES FR GB GR IT LI LU NL SE**

(71) Demandeur: **RHONE-POULENC AGROCHIMIE**
**14-20, rue Pierre Baizet**
**F-69009 Lyon(FR)**

(72) Inventeur: **Axiotis, Georges**
**2 Rue Tête d'Or**
**F-69006 Lyon(FR)**
Inventeur: **Euvrard, Michel**
**33 Route du Mont Thou, Saint Romain au Mont d'Or**
**F-69270 Fontaines Sur Saone(FR)**
Inventeur: **Guigues, François**
**936 Avenue Victor Hugo**
**F-69140 Rilleux(FR)**
Inventeur: **Tadj, Fatemeh**
**17 Place Maréchal Lyautey**
**F-69006 Lyon(FR)**
Inventeur: **Pearson, Christopher John, c/o Research Station of Rhône-Poulenc Agriculture Ltd., Fyfield Road Ongar, Essex CM5 0HW(GB)**

(74) Mandataire: **Brachotte, Charles et al**
**RHONE-POULENC AGROCHIMIE P.I.D. BP 9163**
**F-69263 Lyon Cedex 09(FR)**

(54) **Herbicides dérivés de 2-azolyl nicotinate.**

(57) Composés herbicides de formule :

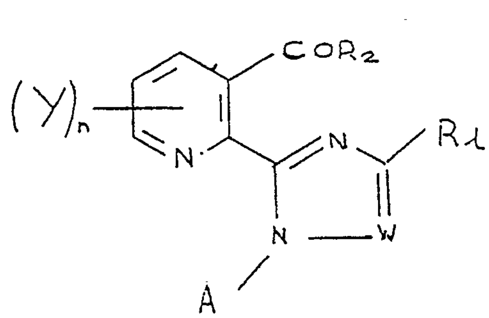

I

dans laquelle
A est l'atome d'hydrogène ou un groupement $R_7SO_2$-
$R_1$ est H ou un groupe hydrocarbyle
$R_2$ est H, un sel, un groupe $X_2R_5$ (ester) $NR_3R_4$ (amide)
$R_7$ est un groupe alkyle, aryle, aralkyle ou $NR_8R_9$
W est -N= ou -$CR_{10}$= où $R_1$, $R_{10}$ ensemble forment un cycle aromatique accolé
Y est un radical alkyle ou alkoxy ou halogène, dialkylamino

EP 0 429 372 A1

n = nombre entier de 1 à 3

L'invention est relative à de nouveaux composés, à leur utilisation à titre d'herbicides notamment sous forme de composition herbicide, à un procédé de contrôle des mauvaises herbes à l'aide de ces composés ou de ces compositions.

On connait par US 4 638 068 des herbicides dérivés de 2-(2-imidazolin-2-yl)-pyridines et quinolines comme l'imazapyr ou 2-(4-isopropyl 4-méthyl 5-oxo 2-imidazolin 2-yl) nicotinique acide (Pest manual - ed $8^{th}$ page 473).

Un objet de la présente invention est de proposer de nouveaux composés utiles en pré ou post-émergence comme herbicides.

Un autre objet de la présente invention est de proposer des composés utiles en pré ou post-émergence comme herbicides antidicotylédones.

Un autre objet de la présente invention est de proposer des composés utiles en pré ou post émergence comme herbicides sélectifs du maïs et de nombreuses autres cultures monocotylédones (blé, orge, riz).

Définition générale de l'invention

Composés de formule I :

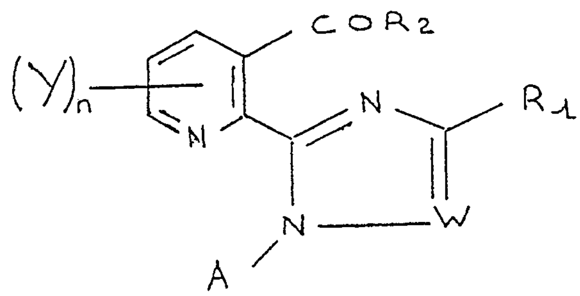

I

dans laquelle :

A est l'atome d'hydrogène ou un groupement $R_7SO_2$-, $R_1$ est un atome d'hydrogène , un radical alkyle éventuellement substitué, un radical cycloalkyle éventuellement substitué, un radical aryle éventuellement substitué , un radical aralkyle éventuellement substitué, les radicaux aryle ou aralkyle pouvant, en outre, comporter dans le cycle 1 à 4 hétéroatomes choisis parmi l'atome d'oxygène, de soufre, d'azote (par exemple furyle, thienyle, pyridyle), $R_2$ est choisi parmi les radicaux de formule $X_1M$, M étant un cation organique ou minéral et $X_1$ étant l'atome d'oxygène ou de soufre , $X_2R_5$, $R_5$ ayant l'une des significations de $R_1$ ou étant un alkyle éventuellement substitué par un groupe alcényle, aloynyle, notamment allyle, propargyl, 2-buténylе, l'alkyle éventuellement substitué par un alcényle ou un alkynyle ne contenant pas de préférence plus de 8 atomes de carbone et $X_2$ étant l'atome d'oxygène ou de soufre, $NR_3R_4$, $R_3$, $R_4$ identiques ou différents étant choisis parmi l'atome d'hydrogène les radicaux alkyle éventuellement substitués, les radicaux cycloalkyle éventuellement substitués, les radicaux aryle éventuellement substitués, les radicaux aralkyle éventuellement substitués ou les radicaux $OR_6$, $R_6$ ayant l'une des significations données pour $R_1$, $R_7$ est un radical alkyle éventuellement substitué , cycloalkyle éventuellement substitué , aryle éventuellement substitué , aralkyle éventuellement substitué , $NR_8R_9$, $R_8$, $R_9$ identiques ou différents ayant l'une des significations données pour $R_1$ ou peuvent former, avec l'atome d'azote auquel ils sont liés, un hétérocyle comportant 1 à 3 hétéroatomes (de préférence comportant 4 à 6 chaînons),

W est un groupe = N - ou = $CR_{10}$ -, $R_{10}$ ayant l'une des significations indiquées pour $R_1$, ou $R_1$, $R_{10}$ ensemble avec les atomes auxquels ils sont rattachés peuvent former un cycle qui, en combinaison avec le noyau imidazole, forment un système aromatique de deux cycles accolés. $R_1$, et $R_{10}$ constituant soit une chaîne à trois atomes dont un de soufre ou d'oxygène et les deux autres de carbone, soit une chaîne à quatre atomes de carbone comportant deux doubles liaisons ou une chaîne à quatre atomes comportant 1 à 3 atomes d'azote et 3 à 1 atomes de carbone, ces chaînes étant éventuellement substituées par un à 4 radicaux $Y_1$ choisis parmi les radicaux alkyle éventuellement substitués, alcoxy ou alkylthio éventuellement substitué, halogène, dialkylamino, deux radicaux choisis parmi les radicaux alcoxy, alkylthio, alkyle ensemble en position 4, 5 ou 5, 6 pouvant former avec les atomes de carbone auxquels ils sont rattachés un cycle aliphatique à 5 ou 6 atomes ayant au plus deux hétéroatomes choisis parmi l'oxygène ou le soufre comme les 1,3-dioxolo [4,5-e]-benzimidazole; 5,6-dihydrofuro[2,3-e]-benzimidazole; 4,5-dihydrofuro[3,2-e]-benzimidazole; 4,5-dihydrothieno[3,2-e]-benzimidazole

Y est un radical alkyle éventuellement substitué, un radical alkoxy ou alkylthio éventuellement substitué, un

atome d'halogène,
en outre lorsque n est égal à 2 ou 3 les radicaux Y adjacents en α et β de l'atome d'azote peuvent ensemble avec les atomes de carbones auxquels ils sont attachés former un groupement noyau phenyl accolé (groupe benzo) ou peuvent former un cycle aliphatique comprenant 5 ou 6 atomes (éventuellement hétérocyclique) et au plus 2 hétéroatomes non adjacents choisis parmi les atomes d'oxygène ou soufre comme les groupes 3,4-dihydropyrano [3, 2- b] pyridine ou 2,3-dihydropyrano [3, 2- b] pyridino ou 2H-3,4 dihydropyran -[3, 2-b] pyridine; 2H-3,4- dihydrothiopyran-[3,2-b] pyridine ou 2,3-dihydrofuro[3,2-b] pyridine,
n est un nombre entier positif ou nul inférieur à 4, étant entendu que lorsque n est supérieur à 1, les groupes Y peuvent être identiques ou différents.

Dans la description qui précède, les radicaux alkyle peuvent être linéaires ou ramifiés et ils ont, de préférence, de 1 à 6 atomes de carbone.

Les radicaux cycloalkyle ont de préférence de 3 à 7 atomes de carbone.

Les radicaux aryle ont, de préférence, entre 6 et 10 atomes de carbone (de préférence phényle).

Les radicaux aralkyle ont, de préférence, entre 7 et 11 atomes de carbone (de préférence benzyle).

Parmi les substituants qui conviennent pour les radicaux précités, on citera notamment les atomes d'halogène (Cl, Br, F) le radical hydroxy, les radicaux $C_1$ - $C_4$ alkyle (mis à part pour les radicaux alkyles précités), les radicaux $C_1$ - $C_4$ alkoxy, les radicaux mono ou polyhalo $C_1$ - $C_4$ alkyle (mis à part pour les radicaux alkyles précités), les radicaux mono ou polyhalo alkoxy, cyano, nitro.

Parmi les cycles aromatiques accolés mentionnés plus haut dans le cas des imidazoles, on peut citer les cycles suivants : benzimidazole, 1 H - thieno[2,3 - d] imidazole, 1 H - thieno [3,4 - d] imidazole, 1 H -- furo [2,3 - d] imidazole, 1 H - furo [3,4 - d] imidazole, 1 H - purine, 1 H - imidazo [4,5 - b] pyrazine, 1 H - imidazo [4,5 - c] pyridine, 1 H imidazo [4,5 - b] pyridine.

Les cations sont choisis, de préférence, parmi les métaux alcalins (Na, K, Li), les sels d'ammonium de formule $NR_{11}R_{12}R_{13}R_{14}$, $R_{11}$, $R_{12}$, $R_{13}$, $R_{14}$ étant choisis parmi l'atome d'hydrogène, les radicaux $C_1$ -$C_6$ alkyle, éventuellement substitués par un radical hydroxy, aralkyle (notamment benzyle) au plus deux des radicaux $R_{11}$ à $R_{14}$ pouvant correspondre au groupe aralkyle.

En raison des propriétés biologiques ou des facilités de synthèse, on préfèrera les composés de formule (I) dans laquelle :

- A est le groupe $R_7SO_2$-, de préférence $R_7$ est $NR_8R_9$, avantageusement, $NMe_2$,
- et/ou n = 0,1,
- et/ou $R_1$ est un radical aryle comportant éventuellement un atome d'azote éventuellement substitué, de préférence $R_1$ est un radical phényle, 2-pyridyle,
- et/ou $R_2$ est le radical hydroxy, allyloxy, propargyloxy ou OM, de préférence M est $NR_{11}R_{12}R_{13}R_{14}$,
- et/ou W est -N= ou -$R_{10}$C=, $R_{10}$ étant choisi parmi l'atome d'hydrogène, le radical phényl ou ter-butyle ou $R_{10}$ forme avec $R_1$, et les deux carbones auxquels ils sont rattachés, un cycle phényle, éventuellement substitué par un ou deux groupes choisis parmi les groupes alcoxy, haloalkoxy ($OCF_3$), alkyle, halogène, haloalkyle.

<u>Procédé de préparation</u>

L'invention concerne également des procédés de préparation des composés de formule (I). Dans l'exposé qui suit, les substituants $R_1$ à $R_{12}$,Y, W ont la même signification que dans la revendication 1.

Les composés de formule (I), dans laquelle A est le groupe $R_7SO_2$- et $R_2$ est un radical $X_2R_5$, avec $R_5$ différent de l'atome d'hydrogène, peuvent être préparés par action d'un chlorure de sulfonyle ou de sulfamoyle de formule $R_7SO_2Cl$ sur un composé de formule (I), dans laquelle A est l'atome d'hydrogène, et $R_2$ a la signification précitée ($X_2R_5$, $R_5$ différents de H), en présence d'un accepteur d'acide tel que le carbonate de potassium, la triéthylamine, le diaza - 1,8 bicyclo [5,9,0] undécène -7, l'hydrure de sodium, de préférence en milieu anhydre, dans un solvant polaire aprotique tel que, par exemple, les éthers comme le THF, les nitriles à une température généralement comprise entre 25° C et le reflux du solvant.

Les composés de formule (I), dans laquelle A est l'atome d'hydrogène, sont préparés par le traitement d'un composé de formule (II)

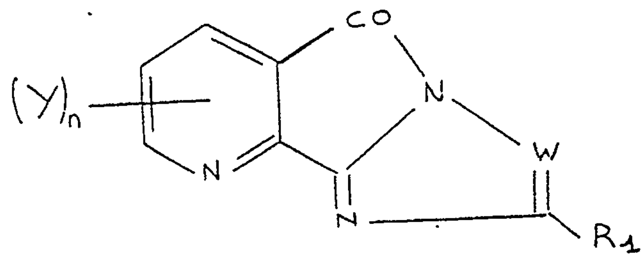

II

avec un alcoolate de métal alcalin ou alcalino-terreux de formule $R_5X_2M'$, $M'$ étant le cation métallique, dans un solvant polaire aprotique à une température généralement comprise entre 0 °C et la température d'ébullition du solvant, ou avec un alcool de formule $R_2OH$ dans un solvant organique polaire en présence d'un accepteur d'acide comme la pyridine ou la triéthylamine.

Un autre procédé, pour obtenir les composés de formule (I) dans laquelle A est l'atome d'hydrogène, où $R_2 \neq X_2H$ consiste à faire réagir un composé de formule (III) :

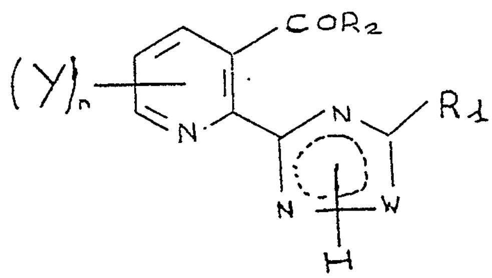

III

où $R_2 = X_2H$
dans un solvant protique tel que le méthanol, en présence d'HCl gazeux avec un alcool de formule $R_5OH$ selon un procédé d'estérification bien connu.

Les composés de formule (II) sont obtenus par action du chlorure de thionyle sur des composés de formule (III), en utilisant le chlorure de thionyle comme solvant et au reflux de celui-ci.

Trois voies permettent d'accéder aux composés de formule (III).

Lorsque W est l'atome d'azote trivalent -N =, les composés de formule (III) peuvent être obtenus en traitant un sel alcalin ou alcalino terreux d'un composé de formule (IV) :

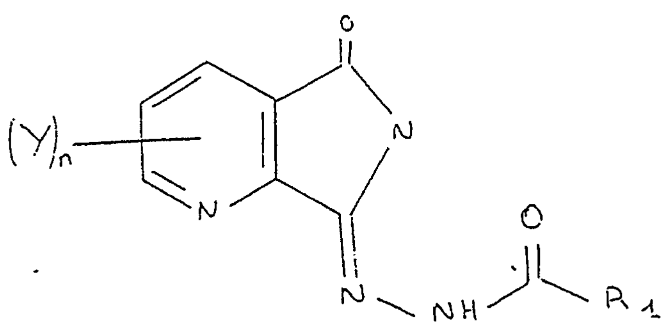

IV

dans l'eau à une température comprise entre 25 °C et 100 °C.

Le sel alcalino-terreux ou alcalin du composé de formule (IV) se prépare par action de la base correspondante sur le composé de formule (IV).

La réaction peut également être effectuée à partir d'un composé de formule (IV) par action d'une base alcaline ou alcalino-terreuse normale 1N telle que la soude ou la potasse à une température comprise entre 25 °C et 100 °C.

Les composés de formule (IV) peuvent être préparés par action d'un hydrazide de formule $R_1$ - $CONHNH_2$ sur un cyanonicotinate de formule (V), décrit dans FR-A-2598708 déposé par RHONE-POULENC AGROCHIMIE, ou dans la référence L.I.M. Spiessens, M.J.O. Anteunis Bull Soc. Chim. Belg. 89,

205, (1980).

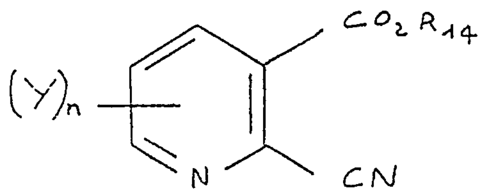

V

dans laquelle $R_{14}$ est un radical alkyle en présence d'un alcoolate de métaux alcalin ou alcalino-terreux dans un solvant polaire protique tel que le méthanol ou l'éthanol à une température comprise entre 25° C et l'ébullition du solvant.

Lorsque W est = $CR_{10}$ -, $R_{10}$ non rattachés à $R_1$, les composés de formule (III) peuvent être obtenus en traitant un composé de formule (VI) :

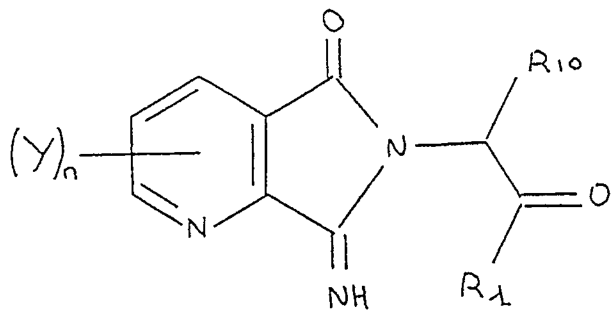

VI

avec une base minérale, la soude ou la potasse, entre 25° C et 100° C en milieu solvant inerte.

Les composés de formule (VI) sont préparés par l'action d'une cétone α - halogénée de formule Br -$HCR_{10}$ - CO - $R_1$ sur un composé de formule (VII) :

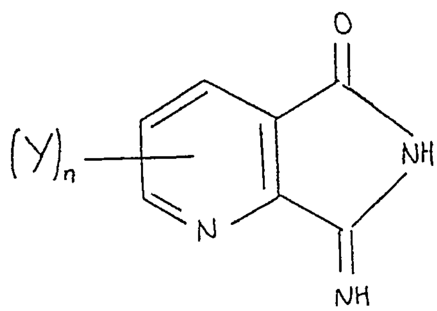

VII

en présence d'un accepteur d'acide comme les carbonates de métaux alcalins, catalysés par des halogénures de métaux alcalins, dans un solvant polaire tel que l'acétone ou le DMF.

Les composés de formule (VII) sont préparés à partir des composés de formule (V) de manière connue dans la littérature (Réf : A.D.Dunn J. Heterocyclic Chem. - 21, 965, (1984)).

Lorsque W est $CR_{10}$ -, $R_{10}$ formant un cycle avec $\overline{R_1}$, les composés de formule (III) peuvent être obtenues par cyclisation des composés de formule (X)

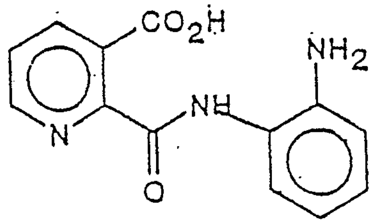

(X)

dans un solvant organique comme l'ethoxyethanol, au reflux.

Le composé de formule (X) est obtenu par réduction d'un composé de formule (XI) en solution aqueuse basique ou organique en présence d'hydrogène et d'un catalyseur d'hydrogénation comme l'oxyde de platine entre 20 et 60° C

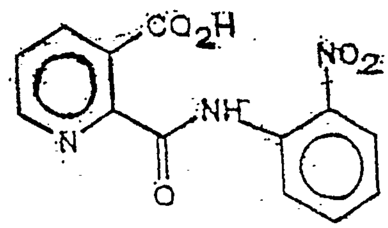

(XI)

Le composé de formule (XI) est obtenue par condensation du composé de formule (VIII) sur une 2-nitroaniline de formule (XII) dans un solvant organique comme le chloroforme ou le tetrahydrofuranne entre 20° C et l'ébullition du solvant

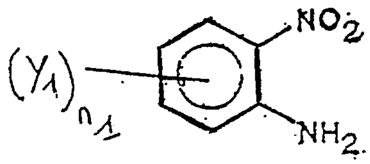

(XII)

Les composés de formule (II) où $R_1$ et $R_{10}$ ensemble forment un cycle aromatique peuvent être obtenus par action d'un anhydride quinolinique de formule (VIII)

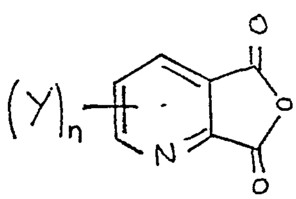

VIII

sur l'orthophénylène de diamine de formule (IX)

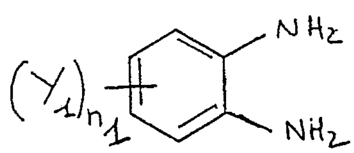

IX

$Y_1$ ayant l'une des significations de Y et $n_1$ étant un nombre entier de 1 à 4, dans un premier temps par chauffage à des températures allant de 110° à 190°, de préférence de 130° à 150°, soit sans solvant, soit

dans un solvant inerte de point d'ébullition élevé tel que xylène ou dichlorobenzène, dans un deuxième temps par traitement du milieu réactionnel par l'anhydride acétique à l'ébullition pendant 1 heure à 3 heures.

Les composés de formule (VIII) et (IX) sont obtenus de la manière connue.

Lorsque l'on veut accéder aux composés de formule (I) dans laquelle $R_2$ est $X_2H$, on hydrolyse par une base minérale par exemple la lithine dans un mélange d'eau et d'alcool tel que le méthanol à une température comprise entre 0° C et 25° C, le composé de formule (I) où $R_2$ est $X_2R_5$, $R_5$ différent de H.

Lorsque l'on veut obtenir les composés de formule (I) dans laquelle $R_2$ est $X_1M$, on fait réagir la base correspondante de manière connue sur la forme acide du composé de formule (I), où $R_2 = X_2H$.

Dans le cas où l'on veut obtenir les composés de formule (I) dans laquelle $R_2$ est $NR_3R_4$, on fait réagir une amine de formule $HNR_3R_4$ avec le composé de formule (II) dans un solvant approprié polaire aprotique. Les composés de formule (I) obtenus sont traités ensuite avec le chlorure de sulfonyle ou de sulfamoyle comme mentionné précédemment.

L'invention a également pour objet les composés de formule (I), où A est l'atome d'hydrogène, (II), (III), (IV), (VI), utilisables comme intermédiaires dans les procédés de préparation des composés de formule (I), dans lesquelles les substituants $R_1$ à $R_{14}$, Y, n, W ont la même signification que dans la formule (I) décrite ci-avant.

D'une manière générale, sans vouloir être lié par une quelconque interprétation scientifique, le déposant pense que c'est la forme acide carboxylique libre ou salifiée qui est active. Aussi, des groupements $COR_2$, non prévus dans la présente description et qui donnent par hydrolyse l'acide carboxylique sous forme libre ou salifiée, font partie de la présente invention.

Exemples de préparation de composés selon l'invention

Exemple 1

Sel d'isopropylamine de 3 -(3 - carboxy 2 - pyridyl) 2 - N,N dimethyl sulfamoyl 5 - phényl 1,2,4 -triazole.

A 1 g d'acide 3 -(3 - carboxy 2 - pyridyl) 2 - N,N -dimethyl sufamoyl 5 - phényl 1,2,4 - triazole, 25 ml d'isopropylamine sont ajoutés à température ambiante. Après une heure d'agitation, un solide précipite. L'amine est évaporée et le solide obtenu est lavé à l'heptane.

F = 180° C.

A partir des acides correspondants dont la synthèse sera décrite ci-après, on a préparé les autres composés suivants de formule (Ia) :

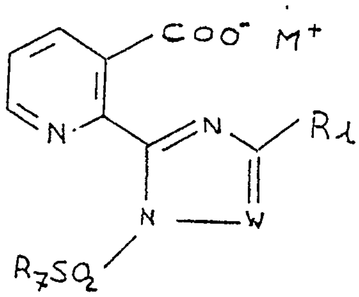

Ia

| EX | W | R1 | R7 | M | F(°C) |
|---|---|---|---|---|---|
| 2 | N | 3,4,5-triCl 2-thienyle | NMe2 | >-NH3 | 168 |
| 3 | N | 2,4-diClPh | NMe2 | >-NH3 | 120 |
| 4 | N | Ph | NEt2 | >-NH3 | 87 |
| 5 | CH | Ph | NMe2 | >-NH3 | 134 |
| 6 | N | Ph | NMe 2 | H3 NCH2 CH2 OH | 77 |

Exemple 7
Acide 3 - (3 - carboxy 2 - pyridyl) 2 - N,N -diméthyl sulfamoyl 5 - phényl 1,2,4 -triazole.

A 1,7 g (0,0044 mole) de 3 - (3 - carbométhoxy 2 -pyridyl) 2 - N,N diméthyl sulfamoyl 5 - phényl 1,2,4 -triazole dissous dans 30 ml de méthanol et maintenu à 0 °C, on ajoute 1 g (0,02 mole) de lithine dissoute dans 10 ml d'eau. Le milieu est ensuite concentré, on amène la solution à pH = 2 par HCl, le précipité blanc obtenu est lavé à l'eau et séché.
m = 0,77 g F = 200 °C

De la même manière, à partir des esters méthyliques correspondants, nous avons obtenu les acides suivants de formule (Ib)

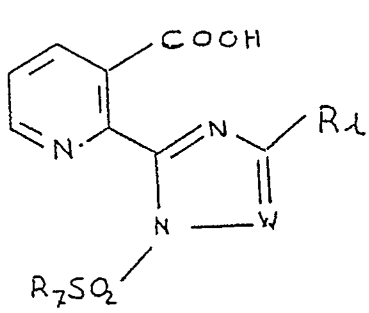

Ib

| EX | W | R1 | R7 | F(°C) |
|---|---|---|---|---|
| 8 | N | 4-OMe Ph | $NMe_2$ | 170 |
| 9 | N | 3-Cl Ph | $NMe_2$ | 205 |
| 10 | N | 3,4,5-triCl 2-thienyle | $NMe_2$ | 230 |
| 11 | N | 2,4 - diClPh | $NMe_2$ | 200 |
| 12 | N | tBu | $NMe_2$ | 169 |
| 13 | N | 4-pyridyle | $NMe_2$ | 197 |
| 14 | N | Ph | $NEt_2$ | 167 |
| 15 | N | Ph | Pyrrolidine | 160 |
| 16 | CH | Ph | $NMe_2$ | 209 |
| 17 | N | 2-Pyridyle | $NME_2$ | 146 |

Exemple 18

3 - (3 - carbométhoxy 2 - pyridyl) 2 - N,N diméthyl sulfamoyl 5 - phényl 1,2,4 - triazole.

A 16,4 g (0,0527 mole) de 3 -(3 - carbométhoxy 2 -pyridyl) 5 - phényl 1,2,4 - triazole dissous dans 50 ml d'acétonitrile anhydre 12,7 g de carbonate de potassium anhydre sont ajoutés. L'ensemble est porté à reflux pendant 2 heures. 8,9 ml (0,062 mole) de chlorure de N,N diméthyl sulfamoyle sont ajoutés au milieu réactionnel qui est refroidi à température ambiante. Après 1 heure à cette température, l'ensemble est porté à reflux.

Un autre équivalent de chlorure de N,N diméthyl sulfamoyle est ajouté, après 5 heures de reflux, au milieu réactionnel.

L'ensemble reste au reflux pendant 10 heures.

Après avoir refroidi le milieu réactionnel, le solide est filtré. Le filtrat est évaporé et chromatographié sur gel de silice (éluant ACEt - Heptane 3/7) pour conduire à 6,1 g de (Ic) (F = 134 °C) et 5,7 g de 3-(3-carbométhoxy 2-pyridyl) 1 N,N diméthyl sulfamoyl 5-phényl 1,2,4-triazole (F= 100 °C).

De la même façon ont été obtenus les composés de formule Ic

Ic

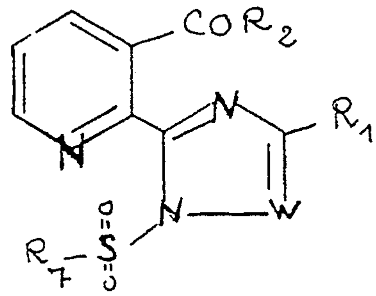

| EX | W | $R_1$ | $R_7$ | $R_2$ | F(°C) |
|---|---|---|---|---|---|
| 19 | N | Ph | $NMe_2$ | OEt | miel |
| 20 | N | benzyl | $NMe_2$ | OMe | miel |
| 21 | N | 3-Cl Ph | $NMe_2$ | OMe | 120 |
| 22 | N | 3-OMe Ph | $NMe_2$ | OMe | 134 |
| 23 | M | 2,4-Cl Ph | $NMe_2$ | OMe | 120 |
| 24 | N | Ph | 2-Cl Ph | OMe | 134 |
| 25 | N | Ph | 2,6-diclPh | OMe | 174 |
| 26 | N | 3,4,5-triCl 2-thienyle | $NMe_2$ | OMe | 180 |
| 27 | N | Ph | $NMe_2$ | $NMe_2$ | 67 |
| 28 | N | Ph | $NEt_2$ | OMe | 97 |

Exemple 29
3 - (3 - carbométhoxy 2 - pyridyl) 5- phényl 1,2,4 triazole.

50 g de 3 - (3 - carboxy 2 - pyridyl) 5 - phényl 1,2,4 - triazole sont dissous dans 800 ml de méthanol. On porte à reflux et on introduit de l'acide chlorhydrique gazeux jusqu'à la fin de la réaction.
Le milieu réactionnel est refroidi, le méthanol est évaporé. A l'huile obtenue, une solution saturée de bicarbonate de sodium est ajoutée jusqu'à neutralité. Un solide précipite. Il est filtré, lavé à l'eau et séché.
m = 49,6 g F = 150 ° C

Exemple 30
3 -(3 - N,N diméthylcarboxamide 2 - pyridyl) 5 -phényl 1,2,4 - triazole.

5,5 g de 3 -(3 - carboxy 2 - pyridyl) 5 - phényl 1,2,4 - triazole (0,022 mole) sont ajoutés à 70 ml de chlorure de thionyle. Après 8 heures de reflux, le chlorure de thionyle est évaporé. On obtient 5,2 g de (II).
F = 288 ° C
Ce dernier est dissous dans 50 ml de THF anhydre et la solution est maintenue à 0 ° C. De la diméthyl amine est introduite dans la solution jusqu'à la fin de la réaction.
Le solvant est évaporé. De l'acide chlorhydrique normal est ajouté jusqu'à neutralité. L'extraction au $CH_2C_{12}$, suivie du séchage et de l'évaporation du solvant, conduit à un miel qui cristallise avec de l'éther diisopropylique. Le solide obtenu est filtré et lavé à l'heptane
m = 5,3 g F = 156 ° C

De la même manière que celle exposée aux exemples 29 et 30, on a obtenu les composés de formule (IIIa) en utilisant les réactifs appropriés :

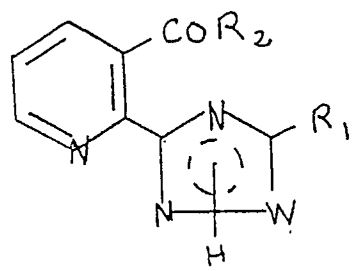

( IIIa )

| EX | W | $R_1$ | $R_2$ | F(°C) |
|---|---|---|---|---|
| 31 | N | 2,4-diCl Ph | OMe | 190 |
| 32 | N | Ph | $O(CH_2)_2$- 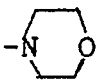 | 171 |
| 33 | N | Ph | ON=CH-C(Me)$_2$ (SMe) | 154 |
| 34 | N | Ph | OEt | 133 |
| 35 | N | Ph | O-< | 145 |
| 36 | N | PH | $SCH_2-CO_2Me$ | 117 |
| 37 | N | 4-OMe Ph | OMe | 204 |
| 38 | CH | Ph | OMe | 126 |
| 39 | N | Ph | $N(CH_3)_3$ | 242 |
| 40 | N | Ph | $NH_2$ | 260 |
| 41 | N | Ph | NHOH | 260 |

Exemple 42

3 - (3 - carboxy 2 - pyridyl) 5 - phényl 1,2,4 -triazole.

93 g de (6 H) 5 - oxo 2 - benzoyl 7 - hydrazo pyrrolo [3,4 - b] pyridine (0,27 mole) sont placés dans 1,8 litre de potasse normale. L'ensemble est porté à reflux pendant 3 heures. Après avoir refroidi le milieu réactionnel, de l'acide chlorhydrique concentré est ajouté jusqu'à pH=2. Le solide précipité est filtré, lavé à l'eau et séché.

m = 57 g F = 242° C

De la même manière, à partir des produits de départ appropriés, sont obtenus les composés de formule (IIIb) suivants (où n = O) :

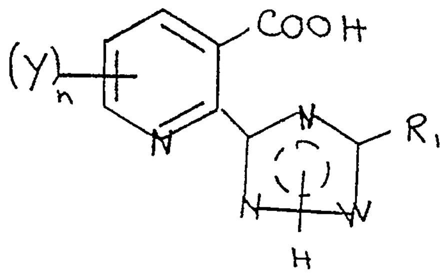

(IIIb)

| EX | Y | W | R1 | F(°C) |
|---|---|---|---|---|
| 43 | H | N | 2,4-diCl Ph | 260 |
| 44 | H | N | 3,4,5-triCl 2-thienyle | 260 |
| 45 | H | N | benzyl | 120 |
| 46 | H | N | 4-pyridyle | >250 |
| 47 | H | N | 4 - OMe Ph | >250 |
| 48 | H | N | 3-Cl Ph | >250 |
| 49 | H | N | 2-Cl Ph | 230 |
| 50 | H | N | 3,5-diCl Ph | >250 |
| 51 | H | N | 2 - pyridyle | 220 |
| 52 | H | N | 2 - $NO_2$ Ph | >250 |
| 53 | H | N | 4 - $CF_3$ Ph | 230 |
| 54 | H | N | 2-Me Ph | 220 |
| 55 | H | N | 4-Me Ph | >250 |

| EX | Y | W | $R_1$ | F(°C) |
| --- | --- | --- | --- | --- |
| 56 | H | N | 2-OMe Ph | 220 |
| 57 | H | N | 4-tBu Ph | 120 |
| 58 | 5-$CH_3$ | N | Ph | 260 |
| 59 | 5-$OCH_3$ | N | Ph | >260 |
| 60 | H | N | $C(CH_3)_3$ | 198 |
| 61 | H | N | H | 230 |
| 62 | H | N | OH | 260 |
| 63 | H | N | 3-$CF_3$ Ph | >250 |
| 64 | H | N | 3,4-OMe Ph | >250 |
| 65 | H | N | 3,5-$CF_3$ Ph | >250 |
| 66 | H | N | 3-OMe Ph | 240 |
| 67 | H | N | styryle | 120 |

| EX | Y | W | R1 | F(°C) |
|---|---|---|---|---|
| 68 | H | N | 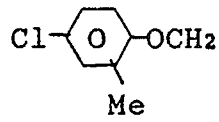 | 195 |
| 69 | H | N | 2-F Ph | >250 |
| 70 | H | N | $(CH_3)_2$-CH | 166 |
| 71 | H | N | benzoyl | 158 |
| 72 | H | N | $CH_3$ | 212 |
| 73 | H | N | 2,5-diCl Ph | >250 |

Exemple 74

2 - (3 - carboxy 2 - pyridyl) 5 - phényl imidazole.

11 g de 5 - oxo 7 - imino 6 - acétophénone pyrrolo (3,4 - b) pyridine (0,041 mole) sont ajoutés à 80 ml de soude normale. Le milieu est dilué avec 50 ml d'eau. L'ensemble est chauffé à reflux pendant 2 heures. Après avoir refroidi le milieu, 100 ml d'acide chlorhydrique normal y sont ajoutés. Un solide précipite , il est filtré, lavé à l'eau et séché.

m = 7,5 g F = 267°C

Exemple 75

Préparation de la 2-(1-N,N diméthylsulfamoyl 2-benzimidazolyl) 3-carboxy pyridine

Une solution de 0,9 g (0,021 mole) d'hydroxyde de lithium hydraté dans 10 ml d'eau est ajoutée à une suspension de 2,5 g (0,007 mole) de 2-(1-diméthyl sulfamoyl 2-benzimidazolyl) 3-carbométhoxy pyridine dans 30 ml de méthanol à température ambiante sous agitation. L'agitation est maintenue jusqu'à dissolution complète du solide, c'est-à-dire pendant trois heures. Le méthanol est éliminé par évaporation sous vide, le résidu repris par 50 ml d'eau et acidifié par 15 ml de solution normale d'acide chlorhydrique. Le précipité blanc formé est filtré, lavé à l'eau et séché.

On obtient : 2,25 g de cristaux blancs fondant à 220°C (Rendement : 93 de la théorie).

De la même façon avec les composés de départ appropriés ont été obtenus les composés suivants :

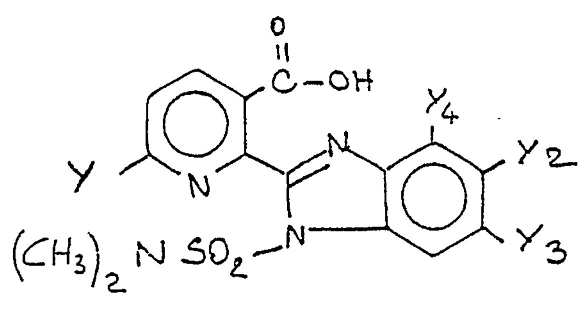

| EX | Y | $Y_4$ | $Y_2$ | $Y_3$ | F(°C) |
|---|---|---|---|---|---|
| 76 | H | H | $CH_3$ | $CH_3$ | 210 |
| 77 | H | H | H | $CF_3$ | 212 |
| 78 | H | H | $CF_3$ | H | 190 |
| 79 | H | Cl | H | H | 190 |
| 80 | ( H | H | Cl | $CF_3$ ) | |
| | ( | | | ) | 210 |
| | ( H | H | $CF_3$ | Cl ) | |
| 81 | H | H | F | F | 210 |
| 82 | H | H | H | CN | 195(d) |
| 83 | H | H | CN | H | 195(d) |
| 84 | H | H | Cl | H | 174 |
| 85 | H | H | H | Cl | 176 |
| 86 | H | $CH_3$ | H | H | 165 |
| 87 | ( H | H | $CH_3$ | H ) | |
| | ( | | | ) | 174 |
| | ( H | H | H | $CH_3$ ) | |
| 88 | H | H | $NO_2$ | H | 198 |
| 89 | H | H | H | $NO_2$ | > 250 |
| 90 | H | $OCH_3$ | H | H | 128-131 |
| 91 | H | $CH_3$ | $CH_3$ | H | 157-158 |
| 92 | H | H | $OCF_3$ | H | 152 |
| 93 | $CH_3$ | H | H | H | 205-206 |

et les deux composés suivants

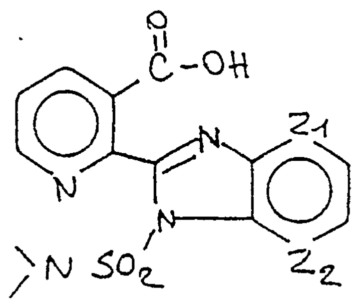

| EX | Z1 | Z2 | F(°C) |
|---|---|---|---|
| 94 | CH | N | 180 |
| 95 | N | CH | 198 |

Exemple 96

Préparation de la 2-(1-N,N diméthylsulfamoyl 2-benzimidazoyl) 3-carbométhoxy pyridine

14,6 ml (0,13 mole) de chlorure de diméthyl sulfamoyle sont ajoutés à une suspension de 8,45 g (0,03 mole) de 2-benzimidazolyl nicotinate de méthyle et 9,2 g (0,066 mole) de carbonate de potassium dans 100 ml d'acétonitrile anhydre.

Le mélange est porté à reflux sous agitation vigoureuse pendant 4 h. Le solvant est évaporé, le résidu est repris par 100 ml d'eau et extrait par 100 ml d'acétate d'éthyle. La phase organique est décantée, séchée et évaporée. Le produit obtenu est purifié par chromatographie sur silice.

On obtient 6,0 g de solide jaune fondant à 180 °C. (Rendement : 50 % de la théorie).

De la même façon ont été obtenus les composés suivants avec les composés de départ appropriés

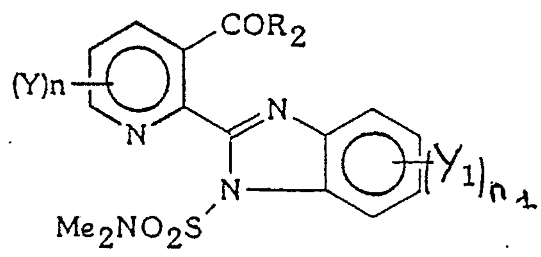

| EX | Y | Y1 | R2 | F(°C) |
|---|---|---|---|---|
| 97 | H | 4,5-di$CH_3$ | $OCH_3$ | 189-191 |
| 98 | H | 5-$OCF_3$ | $OCH_3$ | 135-137,5 |
| 99 | H | H | $OCH_2C{\equiv}CH$ | 187-188,5 |
| 100 | H | H | $OCH_2CF_3$ | huile |
| 101 | H | H | $OCH_2CH{=}CH_2$ | 105-107 |
| 102 | 6-CH3 | H | $OCH_3$ | 165-167 |
| 103 | H | H | $O(CH_2)_5CH_3$ | 68-69,2 |
| 123 | H | H | $OCH_3$ | 180 |
| 124 | H | 5-$CF_3$ | $OCH_3$ | 165 |
| 125 | H | 6-$CF_3$ | $OCH_3$ | 150 |
| 126 | H | 4-Cl | $OCH_3$ | - |
| 127 | H | 5Cl,6-$CF_3$ | $OCH_3$ ) | |
| | | | ) | 210 |
| | H | 5-$CF_3$,6-Cl | $OCH_3$ ) | |
| 128 | H | 5,6-di F | $OCH_3$ | 160 |
| 129 | H | 5-Cl | $OCH_3$ | 158 |
| 130 | H | 6-Cl | $OCH_3$ | 142 |
| 131 | H | 5-$CH_3$ | $OCH_3$ ) | |
| | | | ) | 148 |
| | H | 6-CH3 | $OCH_3$ ) | |
| 132 | H | 5-CN | $OCH_3$ | 173 |
| 133 | H | 6-CN | OCH3 | 214 |
| 134 | H | 4-$CH_3$ | $OCH_3$ | 165 |
| 135 | H | 5-$NO_2$ | $OCH_3$ | 168 |
| 136 | H | 6-$NO_2$ | $OCH_3$ | 150 |

De la même façon avec les produits de départ appropriés sont obtenus les composés suivants

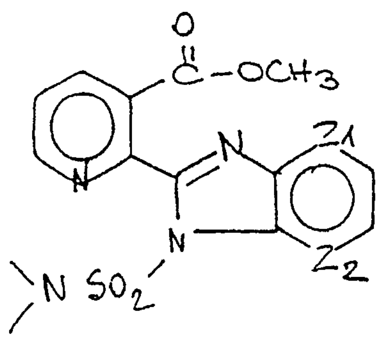

| EX | Z1 | Z2 | F (°C) |
|---|---|---|---|
| 137 | CH | N | 158 |
| 138 | N | CH | 188 |

Exemple 104
Préparation du 2-benzimidazolyl 3-pyridine carboxylate de méthyle,

10,4 g (0,047 mole) de pyrido [2′, 3′ : 3,4] pyrrolo [1,2 - a] benzimidazol-5-one en suspension dans une solution de 0,100 g de potasse dans 30 ml de méthanol sont chauffés à reflux sous agitation pendant 1 heure 30.
Après refroidissement, le précipité formé est filtré, lavé à l'eau et séché.
On obtient : 8,35 g de solide blanc fondant à 200° C. (Rendement 70 % de la théorie).

Exemple 105
Préparation du 2-(4-méthoxy-2-benzimidazolyl) pyridine -3-carboxylate de méthyle

8 ml de triethylamine sont additionné sous agitation à une suspension de pyrido[2′,3′:3,4]pyrrolo[1,2-a]-7-methoxybenzimidazol-5-one (17,4g) dans 260 ml de dichloromethane à température ambiante. Après une demi-heure on ajoute 2,1 ml de méthanol puis la solution est agitée 14 heures, lavée à l'eau, séchée, évaporée. Le produit obtenu est purifié sur colonne pour conduire à 10,5 g d'une gomme.
De la même façon ont été obtenus les composes suivants avec les pyrido[2′3′:3,4]pyrolo[1,2-a]-benzimidazol-5-one et les alcools appropriés :

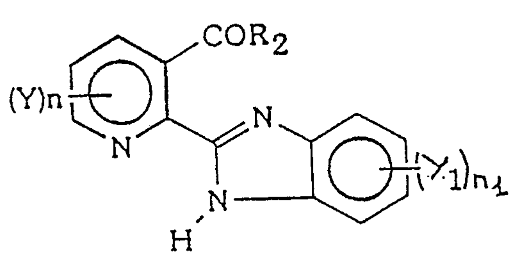

| EX | Y | Y1 | R2 | F(°C) |
|---|---|---|---|---|
| 106 | H | 4,5-di$CH_3$ | $OCH_3$ | 141-143 |
| 107 | H | 5,(6)-$OCF_3$* | $OCH_3$ | 146,5 |
| 108 | H | H | $OCH_2C{\equiv}CH$ | 187,5-189 |
| 109 | H | H | $OCH_2CF_3$ | 177-179 |
| 110 | H | H | $OCH_2CH{=}CH_2$ | 157 |
| 111 | 6$CH_3$ | H | $OCH_3$ | 150-151 |
| 112 | H | H | $O(CH_2)_5CH_3$ | 131-133 |
| 139 | H | H | $OCH_3$ | 200 |
| 140 | H | 5,6-di $CH_3$ | $OCH_3$ | 150 |
| 141 | H | 5-$CF_3$ | $OCH_3$ | 72 |
| 142 | H | 5-Cl,6-$CF_3$ | $OCH_3$ | 180 |
| 143 | H | 4-Cl | $OCH_3$ | 140 |
| 144 | H | 5-Cl | $OCH_3$ | 154 |
| 145 | H | 5,6-di F | $OCH_3$ | - |
| 146 | H | 5-CH3 | OCH3 | 80 |
| 147 | H | 5-CN | $OCH_3$ | > 280 |

(* 50 : 50 mélange d'isomères)

De la même façon avec le réactif de départ approprié est obtenu le composé suivant N° 148 :

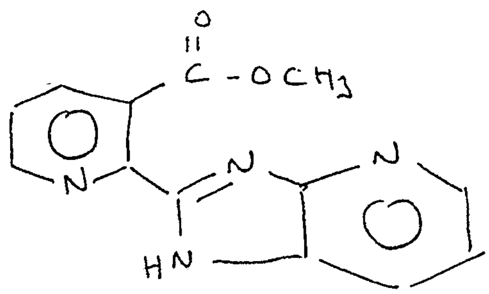

F°C = 230

Exemple 113

Préparation de la pyrido [2', 3' : 3.4] pyrrolo [1,2-a] benzimidazol-5-one,

29,6 g (0,2 mole) d'anhydride quinolinique et 21,5 g (0,2 mole) d'orthophénylène diamine finement broyés et intimement mélangés sont chauffés au bain d'huile à 150° C dans un ballon en rotation autour d'un axe incliné 45 sur l'horizontale pendant 1 heure 15 jusqu'à fin de dégagement d'eau et de gaz.

Après refroidissement, on ajoute 50 ml d'anhydride acétique et on chauffe au reflux pendant 3 heures. Après un nouveau refroidissement, le précipité formé est filtré puis lavé à l'éther diéthylique. On obtient 15,1 g de solide jaune.

Le filtrat est dilué par 100 ml de toluène et neutralisé jusqu'à pH = 6 par agitation avec une solution saturée de bicarbonate de soude. Après décantation, séchage et évaporation, on obtient une huile jaune qui est additionnée d'acétone. Le précipité formé est filtré et séché.

On obtient un deuxième jet de 2,1 g du solide jaune. Au total, on obtient 17,2 g de produit fondant à 225°. (Rendement : 38,9 % de la théorie).
De la même façon ont été préparés les composés suivants: pyrido[2'3':3,4]pyrrolo[1,2-a]-7-methoxybenzimidazol-5-one. F = 180° C (exemple 114) pyrido[2'3':3,4]pyrrolo[1,2-a]-7,8-dimethylbenzimidazol-5-one. F = 196-198° C (exemple 115)
pyrido[2',3':3,4]pyrrolo[1,2-a](8 ou 9)-trifluoromethoxybenzimidazol-5-one. F = 140-151° C (exemple 116)
2-methyl pyrido[2',3':3,4]pyrrolo[1,2-a]benzimidazol-5-one. F = 265,5-268,4° C (exemple 117)

Exemple 118
Préparation du 2-(1-N,N-dimethylsulfamoyl)-2-benzimidazolyl)pyridine-3-carboxylate de benzyltrimethylammonium.

0,48 g d'hydroxyde de N-benzyltrimethylammonium sont ajoutés sous agitation à une solution de 1 g de 2-(1-N,N-dimethylsulfamoyl-2-benzimidazolyl)pyridine-3-acide carboxylique dans 10 ml de méthanol. Après agitation à température ambiante pendant 1,5 heures on évapore le solvant pour donner une huile jaune qui triturée avec l'hexane donne 1,3 g du produit sous la forme d'une crème glacée. De la même façon sont obtenus les sels suivants du même acide.

| EX | Sel (+) |
|---|---|
| 119 | $(CH_3)_2CH\ NH_3$ |
| 120 | $(C_2H_5)_4\ N$ |
| 121 | $C_{12}H_{26}\ NH_3$ |
| 122 | K |

Exemple a
(6 H) 5 - oxo 2 - benzoyl 7 - hydrazo [3,4 -6] pyrrolo pyridine.

50 g de benzhydrazide et 500 g de 2 - cyanonicotinate d'éthyle (0,286 mole) sont ajoutés à une solution de 62,8 g de tertiobutanolate de potassium dans 1,7 litre de méthanol.
Après 2 heures à température ambiante, on chauffe à reflux pendant 3 heures. On obtient un précipité jaune qui, après filtration et lavage au méthanol, conduit à 93 g de solide qui correspond au sel dipotassique de (VI).
Ce composé est caractérisé sous sa forme salifiée. F = 282° C
Suivant ce mode opératoire, ont été obtenus les composés de formule (IVa) suivants (où n = o) :

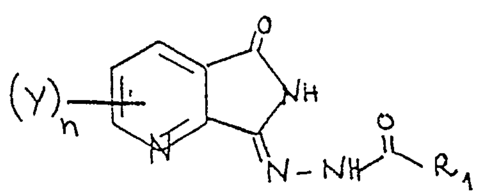

(IVa)

| EX | $R_1$ | F(°C) |
|---|---|---|
| b | 2-Me Ph | |
| c | 4 - tBu Ph | |
| d | 4 - $CF_3$ Ph | |
| e | 2 - $NO_2$ Ph | |
| f | styryl | 283 |
| g | 2-Cl Ph | >250 |
| h | 3-Cl Ph | >250 |
| i | 4-OMe Ph | 200 |
| j | benzyl | 230 |
| k | 3,4,5-triCl 2-thiényle | 260 |
| l | 2,4-diCl Ph | 260 |
| m | 2 - pyridyle | >300 |

| EX | R1 | F(°C) |
|---|---|---|
| n | 3-pyridyle | >300 |
| o | 4-Cl Ph | >260 |
| p | $CH_3$ | 261 |
| q | 4 - pyridyle | >300 |
| r | 4 - Me Ph | |
| s | $H_3C-O$ | 227 |
| t | H | |
| v | $(CH_3)_2CH$ | |
| w | $(CH_3)_3C$ | |
| x | 2,4 - diCl Ph | |

Exemple y

5 - oxo 7 - imino 6 - acétophénone pyrrolo [3,4 -6] pyridine.

1,47 g (0,001 mole) de (VII) est placé dans 100 ml d'acétone. 0,2 g d'iodure de potassium et 3 g de carbonate de potassium sont ajoutés. Après 1/2 heure d'agitation à température ambiante, 2,2 g de bromo acétophénone sont ajoutés. Le milieu réactionnel est agité pendant 16 heures.
Après filtration du solide, le filtrat est évaporé. Le miel obtenu est cristallisé dans l'éther éthylique
m = 1,8 g F = 172° C

L'invention concerne également l'utilisation à titre d'herbicide des composés de formule (I). Comme mauvaises herbes pouvant être contrôlées ou détruites par les composés de formule (I), on peut citer : à titre d'exemples :

Graminées / Cypéracées

Echinochloa crus-galli, Lolium multiflorum, Sorghum halepense, Alopecurus myosuroïdes, Digitaria sanguinalis, Panicum miliaceum, Setaria viridis, Setaria faberie, etc..

Dicotylédones :

Ipomoea purpurea, Abutilon theophrasti, Xanthium pennsylvanicum, Solanum nigrum, Viola Tricolor, Galium aparine, Veronica Spp, Sinapis arvensis, Stellaria media, Polygonum spp, etc..

L'utilisation des composés de formule (I) est la plupart du temps sous forme de composition herbicide comportant un ou plusieurs supports acceptables en agriculture.

En effet, pour leur emploi pratique, les composés selon l'invention sont rarement utilisés seuls. Le plus

souvent ces composés font partie de compositions. Ces compositions, utilisables comme agents herbicides, contiennent comme matière active un composé, selon l'invention, tel que décrit précédemment en mélange avec les supports solides ou liquides, acceptables en agriculture. En particulier, sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement, les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention, un ou plusieurs supports solides ou liquides et éventuellement un ou plusieurs agents tensio-actifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, notamment le butanol, etc...).

L'agent tensio-actif peut être un agent émulsionnant, dispersant ou mouillant de type ionique ou non ionique ou un mélange de tels agents tensio-actifs. On peut citer, par exemple, des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phospates des composés précédents. La présence d'au moins un agent tensio-actif est généralement indispensable lorsque le composé et/ou le support inerte ne sont pas solubles dans l'eau et que l'agent vecteur de l'application est l'eau.

Ainsi donc, les compositions à usage agricole, selon l'invention, peuvent contenir les matières actives selon l'invention dans de très larges limites, allant de $5.10^{-5}$ % à 95 % (en poids). Leur teneur en agent tensio-actif est avantageusement comprise entre 5 et 40 % en poids.

Ces compositions, selon l'invention, sont elles-mêmes sous des formes assez diverses, solides ou liquides.'

Comme formes de compositions solides, on peut citer les poudres pour poudrages (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas).

Les poudres mouillables (ou poudres à pulvériser) sont habituellement préparées de manière à ce qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0 à 10 % d'un ou plusieurs stabilisants et/ou autres additifs, comme des agents de pénétration, des adhésifs ou des agents antimottants, colorants, etc...

Pour obtenir les poudres à pulvériser ou poudres mouillables, on mélange intimement les matières actives dans des mélangeurs appropriés avec les substances additionnelles et on broie avec des moulins ou autres broyeurs appropriés. On obtient par là, des poudres à pulvériser dont la mouillabilité et la mise en suspension sont avantageuses, on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser)

| Exemple F1 : | |
|---|---|
| - matière active (composé n° 1)... | 50,00 % |
| - alcool gras éthoxylé (agent mouillant)... | 2,50 % |
| - phényléthylphénol éthoxylé (agent dispersant)... | 5,00 % |
| - craie (support inerte)... | 42,50 % |

| Exemple F2 | |
|---|---|
| - matière active (composé n° 1)... | 10,00 % |
| - Alcool synthétique oxo de type ramifié, en $C_{13}$ éthoxylé par 8 à 10 oxyde d'éthylène (agent mouillant) ... | 00,75 % |
| - lignosulfonate de calcium neutre (agent dispersant) ... | 12,00 % |
| - carbonate de calcium (charge inerte) q.s.p. | 100 % |

| Exemple F3 : cette poudre mouillable contient les mêmes ingrédients que dans l'exemple précédent, dans les proportions ci-après : | |
|---|---|
| - matière active... | 75,00 % |
| - agent mouillant... | 1,50 % |
| - agent dispersant... | 8,00 % |
| - carbonate de calcium (charge inerte) q.s.p. | 100 % |

| Exemple F4 | |
|---|---|
| - matière active (composé n° 1)... | 90,00 % |
| - alcool gras éthoxylé (agent mouillant)... | 4,00 % |
| - phényléthylphénol éthoxylé (agent dispersant)... | 6,00 % |

| Exemple F5 | |
|---|---|
| - matière active (composé n° 1)... | 50,00 % |
| - mélange de tensio-actifs anioniques et non ioniques (agent mouillant)... | 2,50 % |
| - lignosulfate de sodium (agent dispersant).. | 5,00 % |
| - argile kaolinique (support inerte)... | 42,50 % |

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants

tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle est peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino-terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus.

De préférence, il est obtenu par extrusion en opérant comme indiqué dans les exemples ci-après.

Exemple F6 : Granulés dispersibles

Dans un mélangeur, on mélange 90 % en poids de matière active (composé n° 1) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple F7 : Granulés dispersibles

Dans un mélangeur, on mélange les constituants suivants :

| | |
|---|---|
| - matière active (composé n° 1)... | 75,00 % |
| - agent mouillant (alkylnaphtalène sulfonate de sodium)... | 2,00 % |
| - agent dispersant (polynaphtalène sulfonate de sodium)... | 8,00 % |
| - charge inerte insoluble dans l'eau (kaolin) | 15,00 % |

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Les composés de formule (I) peuvent encore être utilisés sous forme de poudres pour poudrage , on peut aussi utiliser une composition comprenant 50 g de matière active et 950 g de talc, on peut aussi utiliser une composition comprenant 20 g de matière active, 10 g de silice finement divisée et 970 g de talc , on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures. A titre d'exemple, voici la composition de quelques concentrés émulsionnables :

| Exemple F8 : | |
|---|---|
| - matière active... | 400 g/l |
| - dodécylbenzène sulfonate alcalin... | 24 g/l |
| - nonylphénol oxyéthylé à 10 molécules d'oxyde d'éthylène | 16 g/l |
| - cyclohexanone... | 200 g/l |
| - solvant aromatique q.s.p... | 1 litre |

Selon une autre formule de concentré émulsionnable, on utilise :

| Exemple F9 : | |
|---|---|
| - matière active ... | 250 g |
| - huile végétale époxydée... | 25 g |
| - mélange de sulfonate d'alcoylaryle et d'éther de polyglycol et d'alcools gras... | 100 g |
| - diméthylformamide... | 50 g |
| - xylène... | 575 g |

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensio-actifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

| Exemple F10 : | |
|---|---|
| - composé... | 500 g |
| - phosphate de tristyrylphénol polyéthoxyle.. | 50 g |
| - alkylphénol polyéthoxylé... | 50 g |
| - polycarboxylate de sodium... | 20 g |
| - éthylène glycol... | 50 g |
| - huile organopolysiloxanique (antimousse)... | 1 g |
| - polysaccharide... | 1,5 g |
| - eau... | 316,5 g |

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

En ce qui concerne les compositions adaptées au stockage et au transport, elles contiennent plus avantageusement de 0,5 à 95 % (en poids) de substance active.

La présente invention concerne aussi un procédé de désherbage (notamment de zones de culture monocotylédones en particulier maïs, blé, orge, riz) qui consiste à appliquer aux plantes devant être détruites une quantité efficace d'un composé de formule (I).

Lors de l'application à une zone cultivée, la dose d'application devrait être suffisante pour contrôler la naissance des adventices sans causer de dommages substantiels permanents auxdites cultures. Par dose efficace, on entend justement dans ce contexte, la dose qui permet d'obtenir ce résultat.

Les produits et compositions selon l'invention s'appliquent de préférence sur les mauvaises herbes à éliminer lorsque celles-ci présentent un feuillage vert, avantageusement les dicotylédones.

Néanmoins, on pourra utiliser également un procédé de désherbage qui consiste à appliquer une quantité efficace d'un composé de formule (I) sur des zones ou terrains où l'on veut empêcher la pousse ou

le développement de plantes n'ayant pas encore poussé (application de préémergence).

On peut opérer de manière à ce que la culture soit semée avant ou après traitement.

La dose d'application de matière active est généralement comprise entre 1 et 8000 g/ha.

Les exemples ci-dessous illustrent l'invention :

Exemple A - Application herbicide en prélevée des espèces végétales

Dans des pots de 7 x 7 x 8 cm remplis de terre agricole légère, on sème un nombre de graines déterminé en fonction de l'espèce végétale et de la grosseur de la graine.

Les pots sont traités par pulvérisation de bouillie en quantité correspondant à une dose volumique d'application de 500 l/ha et contenant la matière active à la concentration désirée.

Le traitement avec la bouillie est donc effectué sur des graines non recouvertes de terre (on utilise le terme de bouillie pour désigner, en général, les compositions diluées à l'eau, telles qu'on les applique sur les végétaux).

La bouillie utilisée pour le traitement est une solution ou suspension de la matière active dans un mélange acétone/eau en proportions 50/50, en présence de 0,05 en poids de cemulsol NP 10 (agent tensio-actif) constitué d'alkylphénol polyéthoxylé, notamment de nonylphénol polyéthoxylé) et 0,04 % en poids de tween 20 (agent tensio-actif constitué d'un oléate de dérivé polyoxyéthyléné du sorbitol).

Dans le cas d'une suspension, celle-ci est obtenue en mélangeant et broyant les ingrédients dans un microniseur de façon à obtenir une grosseur moyenne de particules inférieure à 40 microns.

Après traitement, on recouvre les graines d'une couche de terre d'environ 3 mm d'épaisseur.

Les pots sont alors placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) des destructions du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage du volume foliaire non détruit par le produit est donc donné par la formule :

$$\frac{[100-D] \times [100-RT]}{100} = A$$

Cette valeur A est transformée en notation de 0 à 5 selon l'échelle suivante :

| Notation | |
|---|---|
| 0 à 10 | 5 (destruction complète) |
| 10 à 30 | 4 |
| 30 à 50 | 3 |
| 50 à 70 | 2 |
| 70 à 90 | 1 |
| 90 à 100 | 0 (pas d'effet) |

Les résultats obtenus sont présentés après l'exemple B pour des doses d'application de 4000 g/ha.

Exemple B - Application herbicide, en postlevée des espèces végétales

Dans des pots de 7 x 7 x 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur et on laisse germer la graine jusqu'à ce qu'elle donne naissance à une plantule au stade convenable. Le stade de traitement pour les graminées est le stade "deuxième feuille en formation ". Le stade de traitement pour les

dicotylédones, est le stade "cotylédons étalés, première feuille vraie en développement".

Les pots sont alors traités par pulvérisation de bouillie en quantité correspondant à une dose volumique d'application de 500 l/ha et contenant la matière active à la concentration désirée.

La bouille a été préparée de la même manière qu'à l'exemple A.

Les pots traités sont ensuite placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage du volume foliaire non détruit par le produit est donc donné par la formule :

$$\frac{[100-D] \times [100-RT]}{100} = A$$

Cette valeur A est transformée en notation de 0 à 5 selon l'échelle suivante :

| Notation | |
|---|---|
| 0 à 10 | 5 (destruction complète) |
| 10 à 30 | 4 |
| 30 à 50 | 3 |
| 50 à 70 | 2 |
| 70 à 90 | 1 |
| 90 à 100 | 0 (pas d'effet) |

Les résultats obtenus sont présentés après le tableau A pour des doses d'application de 4000 g/ha.

Les espèces végétales utilisées dans ces exemples A et B sont :

| ABREVIATIONS | NOM LATIN | NOM FRANCAIS |
|---|---|---|
| ECH | Echinochloa crusgalli | Panisse |
| LOL | Lolium multiflorum | Ray-grass |
| CYP | Cyperus esculentus | Cyperus |
| DIG | Digitaria sanguinalis | Digitaire |
| IPO | Ipomea purpurea | liseron pourpre |
| SIN | Sinapis alba | Moutarde blanche |
| SOL | Solanum nigrum | Morelle noire |
| ABU | Abuliton theophrasti | Abutilon |

| EXEMPLE A : ACTIVITES DE PRE-LEVEES (4 kg/ha) | | | | | | | | |
|---|---|---|---|---|---|---|---|---|
| COMPOSE n° | ECH | LOL | DIG | IPO | SIN | ABU | SOL | CYP |
| 1 | 2 | 2 | 3 | 4 | 5 | 1 | 5 | 5 |
| 2 | 3 | 3 | 4 | 5 | 4 | 0 | 1 | 0 |
| 3 | 5 | 5 | 5 | 5 | 5 | 2 | 3 | 2 |
| 6 | 1 | - | 1 | 4 | 3 | 1 | 5 | 4 |
| 7 | 3 | 2 | 1 | 5 | 3 | 2 | 5 | 5 |
| 8 | 2 | 3 | 1 | 5 | 2 | 3 | 1 | 2 |
| 9 | 4 | 2 | 5 | 5 | 5 | 5 | 5 | 3 |
| 10 | 2 | 1 | 4 | 4 | 4 | 0 | 3 | 0 |
| 14 | 3 | - | 4 | 5 | 1 | 1 | 1 | 2 |
| 24 | 3 | 2 | 4 | 5 | 2 | 1 | 5 | 1 |
| 33 | 4 | 3 | 5 | 4 | 1 | 1 | 2 | 2 |
| 36 | 4 | 2 | 5 | 5 | 2 | 1 | 5 | 1 |
| 37 | 3 | 3 | 3 | 5 | 2 | 5 | 1 | 0 |
| 41 | 4 | 3 | 4 | 5 | 1 | 1 | 0 | 2 |
| 42 | 3 | 2 | - | 5 | 4 | 5 | - | 4 |
| 43 | 4 | 3 | 4 | 3 | 2 | 1 | 1 | 2 |
| 47 | 5 | 3 | 4 | 5 | 2 | 1 | 1 | 3 |
| 48 | 4 | 5 | 4 | 5 | 2 | 5 | 1 | 4 |
| 49 | 4 | 3 | 4 | 3 | 2 | 3 | 2 | 4 |

EXEMPLE A : ACTIVITES DE PRE-LEVEES (4 kg/ha)

| COMPOSE n° | ECH | LOL | DIG | IPO | SIN | ABU | SOL | CYP |
|---|---|---|---|---|---|---|---|---|
| 50 | 4 | 1 | 4 | 5 | 3 | 4 | 0 | 0 |
| 53 | 4 | 3 | 4 | 4 | 4 | 4 | 1 | 0 |
| 54 | 4 | 4 | 4 | 4 | 3 | 4 | 4 | 4 |
| 55 | 4 | 4 | 4 | 3 | 3 | 5 | 5 | 1 |
| 56 | 4 | 3 | 4 | 3 | 3 | 3 | 2 | 4 |
| 57 | 4 | 4 | 4 | 4 | 3 | 4 | 5 | 0 |
| 59 | 3 | 2 | 3 | 5 | 1 | 1 | 0 | 0 |
| 63 | 4 | 2 | 4 | 5 | 5 | 5 | 1 | 3 |
| 64 | 4 | 3 | 4 | 4 | 4 | 1 | 1 | 1 |
| 66 | 3 | 1 | 5 | 4 | 1 | 0 | 0 | 4 |
| 67 | 3 | 3 | 5 | 5 | 2 | 5 | 1 | 0 |
| 69 | - | 3 | 4 | 5 | 2 | 2 | 1 | 3 |
| 73 | 4 | 4 | 4 | 5 | 5 | 4 | 3 | 4 |
| 75 | 1 | - | 2 | 4 | 5 | 2 | 5 | 4 |
| 78 | 3 | - | 1 | 3 | 5 | 0 | 5 | 4 |
| 79 | 1 | - | 0 | 4 | 3 | 1 | 5 | 5 |
| 84 | - | - | 4 | 4 | 5 | 2 | 5 | 5 |
| 87 | 0 | - | 0 | 3 | 5 | 1 | 5 | 3 |
| 121 | 0 | - | 2 | 4 | 4 | 1 | 5 | 0 |

Exemple A(suite) : ACTIVITES DE PRE-LEVEES (1 kg/ha)

| COMPOSE n° | ECH | LOL | DIG | IPO | SIN | ABU | SOL | CYP |
|---|---|---|---|---|---|---|---|---|
| 119 | 0 | - | 0 | 4 | 4 | 1 | 4 | 1 |
| 122 | 0 | - | 1 | 4 | 2 | 1 | 3 | 5 |

| EXEMPLE B : ACTIVITE DE POST-LEVEE (4 kg/ha) | | | | | | |
|---|---|---|---|---|---|---|
| COMPOSE n° | ECH | LOL | DIG | IPO | SIN | SOL |
| 1 | 0 | 0 | 0 | 3 | 5 | 5 |
| 3 | 0 | 0 | 0 | 2 | 2 | 3 |
| 4 | 3 | - | 3 | 2 | 2 | 1 |
| 6 | 0 | - | 0 | 4 | 5 | 5 |
| 7 | 10 | 1 | 1 | 4 | 5 | 5 |
| 9 | 2 | 0 | 2 | 3 | 4 | 3 |
| 12 | 0 | 0 | 0 | 3 | 4 | 4 |
| 15 | 3 | 3 | 3 | 2 | 1 | 1 |
| 24 | 0 | 0 | 0 | 3 | 3 | 3 |
| 42 | 2 | 1 | - | 4 | 4 | - |
| 43 | 4 | 3 | 3 | 4 | 5 | 3 |
| 44 | 2 | 0 | 1 | 3 | 4 | 1 |
| 45 | 3 | 2 | 3 | 3 | 2 | 3 |
| 48 | 3 | 2 | 3 | 3 | 2 | 1 |
| 50 | 3 | 0 | 2 | 3 | 0 | 0 |
| 54 | 2 | 3 | 3 | 0 | 1 | 3 |
| 67 | 0 | 0 | 2 | 3 | 2 | 2 |
| 73 | 3 | 2 | 2 | 3 | 3 | 3 |
| 75 | 0 | - | 0 | 4 | 5 | 5 |
| 84 | 0 | - | 0 | 3 | 5 | 5 |
| 87 | 0 | - | 0 | 4 | 5 | 5 |
| 121 | 3 | - | 0 | 4 | 5 | 5 |
| EXEMPLE B : ACTIVITE DE POST-LEVEE (1 kg/ha) (Suite) | | | | | | |
| COMPOSE n° | ECH | LOL | DIG | IPO | SIN | SOL |
| 119 | 0 | - | 0 | 3 | 4 | 5 |
| 122 | 2 | - | 1 | 4 | 5 | 5 |

Exemple C - Essai de sélectivité en grandes cultures avec application herbicide, en post-levée des espèces végétales.

Dans des pots de 7 x 7 x 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur et on laisse germer la graine jusqu'à ce qu'elle donne naissance à une plantule au stade convenable. Le stade de traitement pour les graminées est le stade "deuxième feuille en formation". Le stade de traitement pour les dicotylédones, est le stade "cotylédons étalés, première feuille vraie en développement".

Les pots sont alors traités par pulvérisation de bouillie en quantité correspondant à une dose volumique d'application de 500 l/ha et contenant la matière active à la concentration désirée.

La bouillie a été préparée de la même manière qu'à l'exemple A.

Les pots traités sont ensuite placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage de volume foliaire non détruit par le produit est donc donné par la formule :

$$\frac{[100-D] \times [100-RT]}{100} = A$$

Cette valeur A est transformée en notation de 0 à 5 selon l'échelle suivante :

| Notation | |
|---|---|
| 0 à 10 | 5 (destruction complète) |
| 10 à 30 | 4 |
| 30 à 50 | 3 |
| 50 à 70 | 2 |
| 70 à 90 | 1 |
| 90 à 100 | 0 (pas d'effet) |

Ainsi, un produit est jugé comme sélectif vis à vis de la culture lorsque la valeur A notée est de 0 ou 1.

Les résultats obtenus sont présentés dans l'exemple C pour des doses d'application comprises entre 0,5 et 4 kg de matière active par hectare selon les produits.

Les espèces végétales utilisées dans cet exemple sont :

| 1) Pour les adventices | | |
|---|---|---|
| ABREVIATIONS | NOM LATIN | NOM FRANCAIS |
| SOL | Solanum nigrum | Morelle noire |
| IPO | Ipomea pupurea | Ipomée |
| SES | Sesbania exaltata | |
| XAN | Xanthium pennsylvanicum | |
| VIO | Viola tricolor | Violette |
| SIN | Sinapis alba | Moutarde blanche |
| POL liseron | Polygonum convolvulus | Renouée |
| CON | Convolvulus arvensis | Liseron des champs |
| CHY | Chrysanthemum segetum | Chrysanthème des moissons |

| 2) Pour les cultures | | |
|---|---|---|
| ABREVIATIONS | NOM LATIN | NOM FRANCAIS |
| TRZ | Triticum aestivum | Blé |
| ZEA | Zea mays | Maïs |
| ORY | Oryza sativa | Riz |

| ESSAI DE SELECTIVITE EN GRANDES CULTURES. ACTIVITE HERBICIDE DE POST LEVEE | | | | | | | | | | | | | |
|---|---|---|---|---|---|---|---|---|---|---|---|---|---|
| Composé n° | dose appli-quée kg/ha | TRZ | ZEA | ORY | SOL | IPO | SES | XAN | VIO | SIN | POL | CON | CHY |
| 6 | 2 | 0 | 0 | 0 | 5 | 5 | 5 | 4 | 4 | - | | | |
| 1 | 1 | 0 | 0 | 0 | 5 | 4 | 5 | 4 | - | 5 | | | |
| 9 | 0,5 | 0 | 2 | 1 | 4 | 3 | 5 | 3 | - | 3 | | | |
| 7 | 2 | 0 | 0 | 0 | 4 | 4 | 5 | 4 | - | 5 | | | |
| 19 | 4 | 0 | 0 | 0 | 5 | 4 | 5 | 2 | - | 5 | | | |
| 75 | 1 | 0 | 1 | 0 | 5 | 5 | 5 | 5 | 4 | | | | |
| 84 | 1 | 1 | 1 | 1 | 5 | 4 | 5 | 3 | 2 | - | 4 | 5 | 5 |
| 79 | 2 | 0 | 0 | 0 | 3 | 4 | 2 | 1 | 1 | - | 3 | 4 | 4 |
| 87 | 2 | 0 | 0 | 0 | 5 | 4 | 3 | 2 | 0 | - | 4 | 5 | 5 |
| 119 | 0,5 | 0 | 0 | 0 | 4 | 3 | 5 | 3 | 3 | - | 5 | 2 | 5 |
| 122 | 1 | 0 | 0 | 0 | 5 | 5 | 5 | 5 | 3 | - | 5 | 5 | 5 |

Comme on peut le voir sur le tableau de résultats de cet exemple C, de nombreux produits présentent une excellente activité antidicotylédones de post-levée tout en montrant une excellente sélectivité pour 1 ou 2 ou 3 des cultures testées : blé, maïs, riz.

Procédé d'utilisation de composés herbicides : Activité herbicide

a) Procédé général

Des quantités appropriées des composés d'essai ont été dissoutes dans de l'acétone pour obtenir des solutions équivalant à des taux d'application de 250, 1000 ou 4000 g de composé d'essai par hectare (g/ha).

Ces solutions ont été appliquées au moyen d'un pulvérisateur usuel d'herbicides de laboratoire en utilisant un jet produit par une fente plate se déplaçant à 2,9 km/h et délivrant l'équivalent de 540 litres de liquide de pulvérisation par hectare (procédé 1) et de 290 litres (procédé 2).

Procédé 1

b) Lutte contre les mauvaises herbes : application en pré-levée

Des graines de mauvaises herbes ont été semées à la surface de compost de mise en pot John Innes N° 1 (7 parties par volume de terre végétale stérilisée, 3 parties en volume de gravier fin) dans des pots en matière plastique de 70 mm de côté et 75 mm de profondeur. Les quantités de graines par pot étaient les suivantes :

| Espèces de mauvaise herbe | Nombre approximatif de graines/pot |
|---|---|
| 1) Mauvaises herbes à feuilles larges | |
| Abutilon theophrasti<br>Sinapis arvensis<br>Chenopodium album<br>Ipomea purpurea | 10<br>20<br>60<br>10 |
| 2) Graminées nuisibles | |
| Avena fatua<br>Echinochloa crus-galli | 15<br>20 |
| 3) Souchets | |
| Cyperus esculentus | 3 |

Les composés de la présente invention ont été appliqués aux graines non recouvertes de la manière décrite dans le paragraphe (a) ci-dessus et ces graines ont été recouvertes avec 25ml de sable liant après pulvérisation. Un seul pot de chaque espèce de mauvaise herbe a été attribué à chaque traitement, en association avec des témoins n'ayant pas subi de pulvérisation et des témoins ayant subi une pulvérisation avec de l'acétone seule. Après traitement, les pots ont été maintenus dans une serre et ont été arrosés par aspersion. L'évaluation visuelle de l'activité de destruction des mauvaises herbes a été effectuée 17 à 20 jours après pulvérisation. Les résultats ont été exprimés par le pourcentage de réduction de croissance ou de mortalité des mauvaises herbes, comparativement aux plantes présentes dans les pots témoins.

c) Lutte contre les mauvaises herbes : application en post-levée

Des espèces de mauvaises herbes ont été cultivées, puis transplantées au stade de jeunes plants de semis dans du compost de mise en pot John Innes N° 1 dans des pots en matière plastique de 70 mm de côté et 75 mm de profondeur, sauf en ce qui concerne Avena fatua, qui a été semée directement dans le pot d'essai et n' a pas été transplantée. Puis les plantes ont été cultivées dans la serre jusqu'à ce qu'elles soient prêtes à subir une pulvérisation avec les composés d'essai. Le nombre de plantes par pot et la croissance de la plante au moment de la pulvérisation étaient les suivantes :

| Espèces de mauvaise herbes | Nombre de plantes par pot | Stades de croissance au moment de la pulvérisation |
|---|---|---|
| 1) Mauvaises herbes à feuilles larges | | |
| Abutilon theophrasti<br>Sinapis arvensis<br>Chenopodium album<br>Ipomea purpurea | 3<br>4<br>4<br>3 | 1-3 feuilles<br>2 feuilles<br>2-4 feuilles<br>1-2 feuilles |
| 2) Graminées nuisibles | | |
| Avena fatua<br>Echinochloa crus-galli | 15<br>4 | 1-2 feuilles<br>2-3 feuilles |
| 3) Souchets | | |
| Cyperus esculentus | 3 | 3 feuilles |

Les composés d'essai ont été appliquées aux plantes de la manière décrite dans le paragraphe (a) ci-dessus. Un seul pot de chaque espèce de mauvaises a été attribué à chaque traitement, en association avec des témoins n'ayant pas subi de pulvérisation et des témoins ayant subi une pulvérisation avec de l'acétone seule. Après pulvérisation, les pots ont été arrossés par aspersion, en commençant 24 heures après pulvérisation. L'évalutation visuelle de la lutte contre la croissance des mauvaises herbes a été effectuée 17 à 20 jours après pulvérisation. Les résultats ont été exprimés en pourcentage de réduction de croissance ou de mortalité des mauvaises herbes, comparativement aux plantes présentes dans les pots témoins.

Procédé 2

d) Lutte contre les mauvaises herbes : pré-levée

Les graines ont été semées dans des pots en matière plastique de 70 mm de côté et 75 mm de profondeur, dans de la terre non stérile provenant de Boarded Barns Farm ou un type de terre similaire provenant d'une source convenable. Les quantités de graines par pot étaient les suivantes :

| Espèces de mauvaise herbes | Nombre approximatif de graines/pot |
|---|---|
| 1) Mauvaises herbes à feuilles larges | |
| Abutilon theophrasti | 10 |
| Amaranthus retroflexus | 20 |
| Galium aparine | 10 |
| Ipomea purpurea | 10 |
| Sinapis arvensis | 15 |
| Xanthium strumarium | 2 |
| 2) Graminées nuisibles | |
| Alpercurus myosuroides | 15 |
| Avena fatua | 10 |
| Echinochloa crus-galli | 15 |
| Setaria viridis | 20 |
| 3) Souchets | |
| Cyperus esculentus | 3 |

| Plantes cultivées | |
|---|---|
| 1) A feuilles larges | |
| Coton | 3 |
| Soja | 3 |
| 2) Graminées | |
| Maïs | 2 |
| Riz | 6 |
| Blé | 6 |

Les composés de la présente invention ont été appliqués à la surface du sol, contenant les semences, de la manière décrite dans le paragraphe (a). Un seul pot de chaque plante cultivée et de chaque mauvaise herbe a été attribué à chaque traitement, en association avec des témoins n'ayant pas subi de pulvérisation et des témoins ayant subi une pulvérisation avec de l'acétone seule. Après traitement, les pots ont été maintenus dans une serre et ont été arrosés par aspersion pendant 3 jours, puis par une sous- irrigation controlée. L'évaluation visuelle des dégats infligés aux plantes cultivées a été effectuée 20 à 24 jours après pulvérisation. Les résultats ont été exprimés en pourcentage de réduction de croissance ou d'endommagement des plantes cultivées ou des mauvaises herbes, comparativement aux plantes présentes dans les pots témoins.

e) Lutte contre les mauvaises herbes : post-levée

Les mauvaises herbes et les plantes cultivées ont été semées directement dans du compost de mise en pot John Innes dans des pots de 75 mm de profondeur et 70 mm de côté, sauf en ce qui concerne l'espèce du genre Amaranthus qui a été repiquée au stade de jeunes plants de semis et transférée dans les pots une semaine avant plulvérisation. Puis les plantes ont été cultivées dans la serre jusqu'à ce qu'elles soient prêtres à subir une pulvérisation avec les composés d'essai. Le nombre de plantes par pot étaient les suivants :

| Espèces de mauvaise herbes | Nombre de plantes par pot | Stade de croissance |
|---|---|---|
| 1) Mauvaises herbes à feuilles larges | | |
| Abutilon theophrasti | 3 | 1-2 feuilles |
| Ammaranthus retroflexus | 4 | 1-2 feuilles |
| Galium aparine verticille | 3 | 1er |
| Ipomea purpurea | 3 | 1-2 feuilles |
| Sinapis arvensis | 4 | 2 feuilles |
| Xanthium strumarium | 1 | 2-3 feuilles |
| 2) Graminées nuisibles | | |
| Alopecurus myosuroides | 8-12 | 1-2 feuilles |
| Avena fatua | 12-18 | 1-2 feuilles |
| Echinochloa crus-galli | 4 | 2-3 feuilles |
| Setaria viridis | 15-25 | 1-2 feuilles |
| 3) Souchets | | |
| Cyperus esculentus | 3 | 3 feuilles |

| Plantes cultivées | Nombre de plantes par pot | Stade de croissance |
|---|---|---|
| 1) A feuilles larges | | |
| Coton<br>Soja | 2<br>2 | 1 feuille<br>2 feuilles (1er groupe de 3 feuilles) |
| 2) Graminées | | |
| Maïs<br>Riz<br>Blé | 2<br>4<br>5 | 2-3 feuilles<br>2-3 feuilles<br>2-3 feuilles |

Les composés d'essai ont été appliqués aux plantes de la manière décrite dans le paragraphe (a). Un seul pot de chaque espèce de plante cultivée et de chaque espèce de mauvaise herbe a été attribué à chaque traitement, en association avec des témoins n'ayant pas subi de pulvérisation et des témoins ayant subi une pulvérisation avec de l'acétone seule.

Après traitement, les pots ont été maintenus dans une serre et ont été arrosés une fois par aspersion au bout de 24 heures, puis par sous -irrigation contrôlée. L'évaluation visuelle des dégâts infligés aux plantes cultivées et de la destruction des mauvaises herbes a été effectué 20 à 24 jours après pulvérisation. Les résultats ont été exprimés en pourcentage de réduction de croissance ou d'endommagement des plantes cultivées ou des mauvaises herbes, comparativement aux plantes présentes dans les pots témoins.

Lorsqu'ils ont été appliqués en pré-levée à 4000 g/ha (procédé 1B), les composés (101, 92, 100) ont provoqué une réduction d'au moins 90 % de la croissance d'une ou plusieurs des espèces de mauvaises herbes.

Lorsqu'ils ont été appliqués en post-levée à 4000 h/ha (procédé 1C), les composés 101, 92, 100) ont provoqué de nouveau une réduction d'au moins 90 % de la croissance d'une ou plusieurs des espèces de mauvaises herbes.

Lorsqu'ils ont été appliqués en pré-levée à 4000 g/ha (procédé 2D), les composés (118, 91, 90, 103, 93, 99) ont provoqué une réduction d'au moins 90 % de la croissance d'une ou plusieurs des espèces de mauvaises herbes.

Lorsqu'ils ont été appliqués en post-levée à 4000 g/ha (procédé 1E), les composés (118, 91, 90, 103, 93, 99) ont provoqué une destruction d'au moins 90 % d'une ou plusieurs des espèces de mauvaises herbes.

## Revendications

1 - Composés de formule I

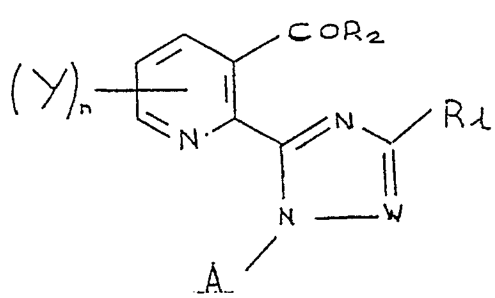

I

dans laquelle :

A est l'atome d'hydrogène ou un groupement $R_7SO_2$-, $R_1$ est un atome d'hydrogène, un radical alkyle éventuellement substitué , un radical cycloalxyle éventuellement substitué, un radical aryle éventuellement

substitué, un radical aralkyle éventuellement substitué, les radicaux aryle ou aralkyle pouvant, en outre, comporter dans le cycle 1 à 4 hétéroatomes choisis parmi l'atome d'oxygène, de soufre, d'azote (par exemple furyle, thiényle, pyridyle), $R_2$ est choisi parmi les radicaux de formule $X_1M$, M étant un cation organique ou minéral et $X_1$ étant l'atome d'oxygène ou de soufre , $X_2R_5$, $R_5$ ayant l'une des significations de $R_1$ ou étant un alkyle éventuellement substitué par un groupe alcényle, alcynyle, notamment allyle, propargyle, 2-butènyle, l'alkyle éventuellement substitué par un alcényle ou un alcynyle ne comprenant pas, de préférence, plus de 8 atomes de carbone et $X_2$ étant l'atome d'oxygène ou de soufre , $NR_3R_4$, $R_3$, $R_4$ identiques ou différents étant choisis parmi l'atome d'hydrogène les radicaux alkyles éventuellement substitués, les radicaux cycloalkyle éventuellement substitués, les radicaux aryles éventuellement substitués, les radicaux aralkyle éventuellement substitués ou les radicaux $OR_6$, $R_6$ ayant l'une des significations données pour $R_1$,

$R_7$ est un radical alkyle éventuellement substitué , cycloalkyle éventuellement substitué , aryle éventuellement substitué, aralkyle éventuellement substitué , $NR_8R_9$, $R_8$, $R_9$ identiques ou différents ayant l'une des significations données pour $R_1$ ou peuvent former, avec l'atome d'azote auquel ils sont liés, un hétérocyle comportant 1 à 3 hétéroatomes (de préférence comportant 4 à 6 chaînons),

W est un groupe = N - ou = $CR_{10}$ -, $R_{10}$ ayant l'une des significations indiquées pour $R_1$, où $R_1$, $R_{10}$ ensemble avec les atomes auxquels ils sont rattachés peuvent former un cycle qui, en combinaison avec le noyau imidasole, forment un système aromatique de deux cycles accolés, $R_1$, et $R_{10}$ constituant soit une chaîne à trois atomes dont un de soufre ou d'oxygène et les deux autres de carbone, soit une chaîne à quatre atomes de carbone comportant deux doubles liaisons ou une chaîne à quatre atomes comportant 1 à 3 atomes d'azote et 3 à 1 atomes de carbone, ces chaînes étant éventuellement substituées par un à 4 radicaux Y1 choisis parmi les radicaux alkyle éventuellement substitués, alcoxy éventuellement substitué, halogène, dialkylamino, 2 radicaux choisis parmi les radicaux alcoxy, alkylthio, alkyl ensemble en position 4,5 ou 5,6 pouvant former avec les atomes de carbones auxquels ils sont rattachés un cycle aliphatique à 5 ou 6 atomes ayant au plus 2 hétéroatomes choisis parmi l'oxygène ou le soufre,

Y est un radical alkyle éventuellement substitué, un radical alkoxy ou alkylthio éventuellement substitué, un atome d'halogène, lesdits radicaux Y lorsque n est égal à 2 ou 3 et lorsque ils sont en position adjacente en $\alpha$ et $\beta$ de l'atome d'azote pouvant, en outre, ensemble avec les atomes de carbone auxquels ils sont attachés former un noyau phenyl accolé (groupe benzo) ou peuvent former un cycle aliphatique (éventuellement hétérocyclique) comprenant 5 ou 6 atomes dont au plus 2 hétéroatomes non adjacents choisis parmi les atomes d'oxygène ou de soufre, n est un nombre entier positif ou nul inférieur à 4, étant entendu que lorsque n est supérieur à 1, les groupes Y peuvent être identiques ou différents.

2 - Composés selon la revendication 1, caractérisé en ce que les radicaux alkyles ont 1 à 6 atomes de carbone.

3 - Composés selon la revendication 1, caractérisés en ce que les radicaux cycloalkyles ont de préférence de 3 à 7 atomes de carbone.

4 - Composés selon la revendication 1, caractérisés en ce que les radicaux aryles ont de 6 à 10 atomes de carbone (de préférence phényle).

5 - Composés selon la revendication 1, caractérisés en ce que les radicaux aralkyles ont entre 7 et 11 atomes de carbone (de préférence benzyle).

6 - Composés selon la revendication 1, caractérisés en ce que les substituants qui conviennent sont les atomes d'halogène (Cl, Br, F) le radical hydroxy, les radicaux $C_1$ - $C_4$ alkyle (mis à part pour les radicaux alkyles précités, les radicaux $C_1$ - $C_4$ alkoxy, les radicaux mono ou polyhalo $C_1$ - $C_4$ alkyle (mis à part pour les radicaux alkyles précités), les radicaux mono ou polyhalo alkoxy, cyano, nitro.

7 - Composés selon la revendication 1, caractérisés en ce que les cations sont choisis parmi les métaux alcalins (Na, K, Li), les radicaux ammoniums de formule $NR_{11}R_{12}R_{13}R_{14}$, $R_{11}$, $R_{12}$, $R_{13}$, $R_{14}$ étant choisis parmi l'atome d'hydrogène, les radicaux $C_1$ -$C_6$ alkyle éventuellement substitués par un radical hydroxy, aralkyle (notamment benzyle) au plus deux des radicaux $R_{11}$ à $R_{14}$ pouvant correspondre au groupe aralkyle.

8 - Composés selon la revendication 1, caractérisés en ce que :

- A est le groupe $R_7SO_2$-, $R_7$ est de préférence $NR_8R_9$, avantageusement $NMe_2$,
- et/ou n = 0,1
- et/ou $R_1$ est un radical aryle éventuellement substitué, de préférence $R_1$ est un radical phényle, 2-pyridyle,
- et/ou $R_2$ est le radical hydroxy, allyloxy, propargyloxy ou OM, de préférence M est $NR_{11}R_{12}R_{13}R_{14}$ ,
- et/ou W est -N= ou -$R_{10}$C=, $R_{10}$ étant choisi parmi l'atome d'hydrogène, le radical phényle ou ter-butyle où $R_{10}$ forme avec $R_1$ et les deux atomes de carbone auxquels ils sont rattachés un cycle phényle éventuellement substitué par un ou deux groupe choisi parmi les groupes alkoxy, haloalcoxy, alkyle,

halogène, haloalkyle.

9 - Utilisation des composés selon l'une des revendications 1 à 8 à titre d'herbicide notamment sous la forme d'une composition herbicide comportant en outre un support inerte.

10 - Composition herbicide comportant 0,05 à 95 % en poids d'une manière active selon l'une des revendications 1 à 8 en combinaison avec les supports solides ou liquides acceptables en agriculture et les agents tensio-actifs acceptables en agriculture.

11 - Procédé de désherbage (notamment de zones de culture monocotylédones telles que le maïs, blé, orge, riz) qui consiste à appliquer aux plantes devant être détruites une quantité efficace d'un composé de formule (I) selon l'une des revendications 1 à 8, de préférence en post-émergence.

12 - Procédé de préparation des composés de formule (I) selon la revendication 1, caractérisé en ce qu'ils sont obtenus:

- 1) dans le cas où A est le groupe $R_7$ $SO_2$- et par action d'un chlorure de sulfonyle ou de sulfamoyle de formule $R_7SO_2Cl$ sur un composé de formule (I) dans laquelle A est l'atome d'hydrogène, en présence d'un accepteur d'acide.

- 2) dans le cas où A est l'atome d'hydrogène et $R_2$ est un radical $X_2R_5$ avec $R_5$ # H, par action d'un composé de formule (II), $R_2$ est un radical $X_2R_5$, avec $R_5$ différent de l'atome d'hydrogène :

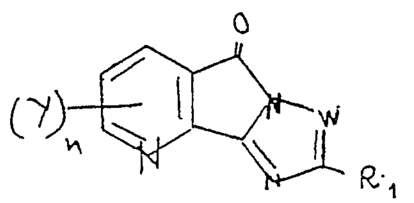

( II )

avec un alcoolate de métal alcalin ou alcalino-terreux de formule $R_5X_2M'$, $M'$ étant le cation métallique, ou avec un alcool de formule $R_2OH$ en présence d'un accepteur d'acide..

- 3) dans le cas où A est l'atome d'hydrogène avec $R_5$ différent de l'atome d'hydrogène, par estérification d'un composé de formule (III) où $R_2$ est un radical $X_2H$, avec un alcool de formule $R_5OH$.

- 4) dans le cas où $R_2$ est $X_2H$ et A est le groupe $R_7SO_2$ par hydrolyse du composé de formule (I) où $R_2$ est $X_2R_5$, $R_5$ différent de H.

- 5) dans le cas où $X_2$ est $X_1M$ et A est le groupe $R_7SO_2$ par action de la base correspondante sur la forme acide du composé de formule (I).

- 6) dans le cas où $R_2$ est $NR_3R_4$ on fait réagir une amine de formule $HNR_3R_4$ avec le composé de formule (II) puis traitement éventuel avec le composé de formule $R_7SO_2Cl$.

- 7) Les composés de formule (II) où $R_1$ et $R_{10}$ ensemble forment un cycle aromatique peuvent être obtenus par action d'un anhydride quinolinique de formule (VIII)

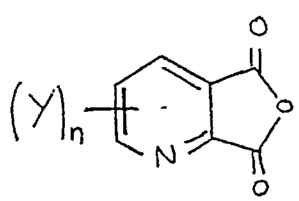

VIII

sur l'orthophénylène de diamine de formule (IX)

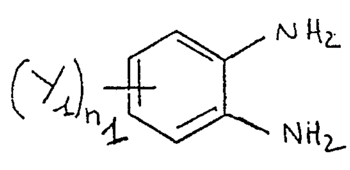

IX

Y, $Y_1$, n, n1 ayant l'une des significations indiquées à la revendication 1.

- 8) Lorsque W est = $CR_{10}$-, $R_{10}$ formant un cycle avec $R_1$, les composés de formule (III) peuvent être

obtenus par cyclisation des composés de formule (X) :

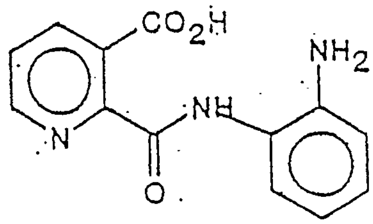

(X)

13 - Composés utiles notamment dans le procédé selon la revendication 12 de formule (II).

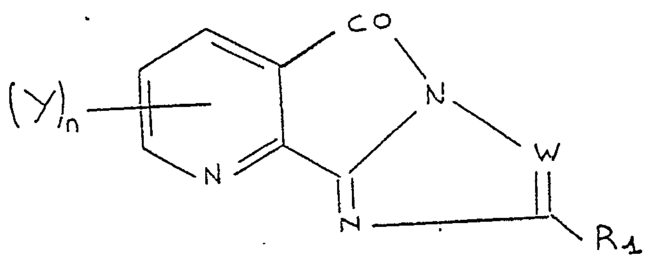

II

Claims for the following Contracting States: ES, GR

1 - Composition herbicide comportant un composé de formule

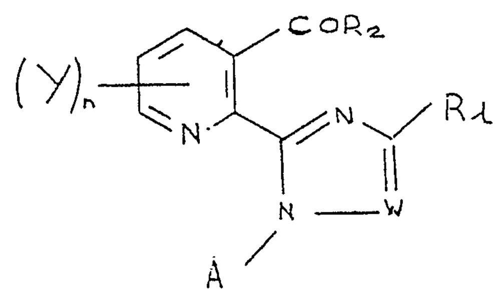

I

dans laquelle :

A est l'atome d'hydrogène ou un groupement $R_7SO_2$-, $R_1$ est un atome d'hydrogène , un radical alkyle éventuellement substitué , un radical cycloalkyle éventuellement substitué, un radical aryle éventuellement substitué , un radical aralkyle éventuellement substitué, les radicaux aryle ou aralkyle pouvant, en outre, comporter dans le cycle 1 à 4 hétéroatomes choisis parmi l'atome d'oxygène, de soufre, d'azote (par exemple furyle, thiényle, pyridyle), $R_2$ est choisi parmi les radicaux de formule $X_1M$, M étant un cation organique ou minéral et $X_1$ étant l'atome d'oxygène ou de soufre , $X_2R_5$, $R_5$ ayant l'une des significations de $R_1$ ou étant un alkyle éventuellement substitué par un groupe alcényle, alcynyle, notamment allyle, propargyle, 2-butènyle, l'alkyle éventuellement substitué par un alcényle ou un alcynyle ne comprenant pas, de préférence, plus de 8 atomes de carbone et $X_2$ étant l'atome d'oxygène ou de soufre , $NR_3R_4$, $R_3$, $R_4$ identiques ou différents étant choisis parmi l'atome d'hydrogène les radicaux alkyles éventuellement substitués, les radicaux cycloalkyle éventuellement substitués, les radicaux aryles éventuellement substitués, les radicaux aralkyle éventuellement substitués ou les radicaux $OR_6$, $R_6$ ayant l'une des significations données pour $R_1$,

$R_7$ est un radical alkyle éventuellement substitué , cycloalkyle éventuellement substitué , aryle éventuellement substitué , aralkyle éventuellement substitué , $NR_8R_9$, $R_8$, $R_9$ identiques ou différents ayant l'une des significations données pour $R_1$ ou peuvent former, avec l'atome d'azote auquel ils sont liés, un hétérocycle comportant 1 à 3 hétéroatomes (de préférence comportant 4 à 6 chaînons),

W est un groupe = N - ou = $CR_{10}$ -, $R_{10}$ ayant l'une des significations indiquées pour $R_1$, où $R_1$, $R_{10}$

ensemble avec les atomes auxquels ils sont rattachés peuvent former un cycle qui, en combinaison avec le noyau imidazole, forment un système aromatique de deux cycles accolés, $R_1$, et $R_{10}$ constituant soit une chaîne à trois atomes dont un de soufre ou d'oxygène et les deux autres de carbone, soit une chaîne à quatre atomes de carbone comportant deux doubles liaisons ou une chaîne à quatre atomes comportant 1 à 3 atomes d'azote et 3 à 1 atomes de carbone, ces chaînes étant éventuellement substituées par un à 4 radicaux Y1 choisis parmi les radicaux alkyle éventuellement substitués, alcoxy éventuellement substitué, halogène, dialkylamino, 2 radicaux choisis parmi les radicaux alcoxy, alkylthio, alkyl ensemble en position 4,5 ou 5,6 pouvant former avec les atomes de carbones auxquels ils sont rattachés un cycle aliphatique à 5 ou 6 atomes ayant au plus 2 hétéroatomes choisis parmi l'oxygène ou le soufre,

Y est un radical alkyle éventuellement substitué, un radical alkoxy ou alkylthio éventuellement substitué, un atome d'halogène, lesdits radicaux Y lorsque n est égal à 2 ou 3 et lorsque ils sont en position adjacente en $\alpha$ et $\beta$ de l'atome d'azote pouvant, en outre, ensemble avec les atomes de carbone auxquels ils sont attachés former un noyau phenyl accolé (groupe benzo) ou peuvent former un cycle aliphatique (éventuellement hétérocyclique) comprenant 5 ou 6 atomes dont au plus 2 hétéroatomes non adjacents choisis parmi les atomes d'oxygène ou de soufre, n est un nombre entier positif ou nul inférieur à 4, étant entendu que lorsque n est supérieur à 1, les groupes Y peuvent être identiques ou différents.

2 - Composition selon la revendication 1, caractérisé en ce que les radicaux alkyles ont 1 à 6 atomes de carbone.

3 - Composition selon la revendication 1, caractérisés en ce que les radicaux cycloalkyles ont de préférence de 3 à 7 atomes de carbone.

4 - Composition selon la revendication 1, caractérisés en ce que les radicaux aryles ont de 6 à 10 atomes de carbone (de préférence phényle).

5 - Composition selon la revendication 1, caractérisés en ce que les radicaux aralkyles ont entre 7 et 11 atomes de carbone (de préférence benzyle).

6 - Composition selon la revendication 1, caractérisés en ce que les substituants qui conviennent sont les atomes d'halogène (Cl, Br, F) le radical hydroxy, les radicaux $C_1$ - $C_4$ alkyle (mis à part pour les radicaux alkyles précités, les radicaux $C_1$ - $C_4$ alkoxy, les radicaux mono ou polyhalo $C_1$ - $C_4$ alkyle (mis à part pour les radicaux alkyles précités), les radicaux mono ou polyhalo alkoxy, cyano, nitro.

7 - Composition selon la revendication 1, caractérisés en ce que les cations sont choisis parmi les métaux alcalins (Na, K, Li), les radicaux ammoniums de formule $NR_{11}R_{12}R_{13}R_{14}$, $R_{11}$, $R_{12}$, $R_{13}$, $R_{14}$ étant choisis parmi l'atome d'hydrogène, les radicaux $C_1$ - $C_6$ alkyle éventuellement substitués par un radical hydroxy, aralkyle (notamment benzyle) au plus deux des radicaux $R_{11}$ à $R_{14}$ pouvant correspondre au groupe aralkyle.

8 - Composition selon la revendication 1, caractérisés en ce que :

- A est le groupe $R_7SO_2$-, $R_7$ est de préférence $NR_8R_9$, avantageusement $NMe_2$,
- et/ou n = 0,1
- et/ou $R_1$ est un radical aryle éventuellement substitué, de préférence $R_1$ est un radical phényle, 2-pyridyle,
- et/ou $R_2$ est le radical hydroxy, allyloxy, propargyloxy ou OM, de préférence M est $NR_{11}R_{12}R_{13}R_{14}$ ,
- et/ou W est -N= ou -$R_{10}$C=, $R_{10}$ étant choisi parmi l'atome d'hydrogène, le radical phényle ou ter-butyle où $R_{10}$ forme avec $R_1$ et les deux atomes de carbone auxquels ils sont rattachés un cycle phényle éventuellement substitué par un ou deux groupe choisi parmi les groupes alkoxy, haloalcoxy, alkyle, halogène, haloalkyle.

9 - Utilisation des composés selon l'une des revendications 1 à 8 à titre d'herbicide notamment sous la forme d'une composition herbicide comportant en outre un support inerte.

10 - Composition herbicide comportant 0,05 à 95 % en poids d'une manière active selon l'une des revendications 1 à 8 en combinaison avec les supports solides ou liquides acceptables en agriculture et les agents tensio-actifs acceptables en agriculture.

11 - Procédé de désherbage (notamment de zones de culture monocotylédones telles que le maïs, blé, orge, riz) qui consiste à appliquer aux plantes devant être détruites une quantité efficace d'un composé de formule (I) selon l'une des revendications 1 à 8, de préférence en post-émergence.

12 - Procédé de préparation des composés de formule (I) selon la revendication 1, caractérisé en ce qu'ils sont obtenus :

- 1) dans le cas où A est le groupe $R_7$ $SO_2$- et par action d'un chlorure de sulfonyle ou de sulfamoyle de formule $R_7SO_2Cl$ sur un composé de formule (I) dans laquelle A est l'atome d'hydrogène, en présence d'un accepteur d'acide.
- 2) dans le cas où A est l'atome d'hydrogène et $R_2$ est un radical $X_2R_5$ avec $R_5$ # H, par action d'un composé de formule (II), $R_2$ est un radical $X_2R_5$, avec $R_5$ différent de l'atome d'hydrogène :

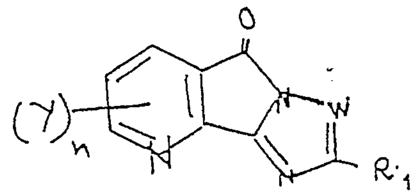

(II)

- avec un alcoolate de métal alcalin ou alcalino-terreux de formule $R_5X_2M'$, $M'$ étant le cation métallique, ou avec un alcool de formule $R_2OH$ en présence d'un accepteur d'acide..

- 3) dans le cas où A est l'atome d'hydrogène avec $R_5$ différent de l'atome d'hydrogène, par estérification d'un composé de formule (III) où $R_2$ est un radical $X_2H$, avec un alcool de formule $R_5OH$.

- 4) dans le cas où $R_2$ est $X_2H$ et A est le groupe $R_7SO_2$ par hydrolyse du composé de formule (I) où $R_2$ est $X_2R_5$, $R_5$ différent de H.

- 5) dans le cas où $X_2$ est $X_1M$ et A est le groupe $R_7SO_2$ par action de la base correspondante sur la forme acide du composé de formule (I).

- 6) dans le cas où $R_2$ est $NR_3R_4$ on fait réagir une amine de formule $HNR_3R_4$ avec le composé de formule (II) puis traitement éventuel avec le composé de formule $R_7SO_2Cl$.

- 7) Les compositions de formule (II) où $R_1$ et $R_{10}$ ensemble forment un cycle aromatique peuvent être obtenus par action d'un anhydride quinolinique de formule (VII)

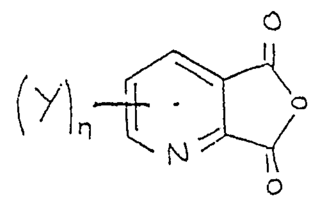

VIII

sur l'orthophénylène de diamine de formule (IX)

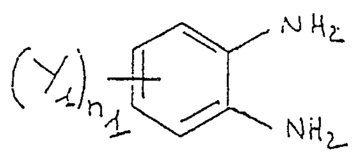

IX

Y, $Y_1$, n, n1 ayant l'une des significations indiquées à la revendication 1.

- 8) Lorsque W est " $CR_{10}$-, $R_{10}$ formant un cycle avec $R_1$, les composés de formule (III) peuvent être obtenus par cyclisation des composés de formule (X) :

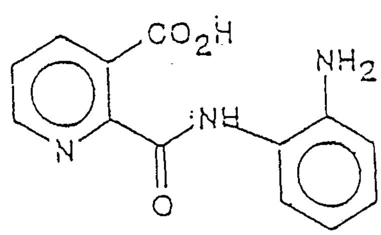

(X)

Office européen des brevets

**RAPPORT PARTIEL DE RECHERCHE EUROPEENNE**
qui selon la règle 45 de la Convention sur le brevet européen est considéré, aux fins de la procédure ultérieure, comme le rapport de recherche européenne

Numéro de la demande: EP 90 42 0452

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 298 029 (CIBA GEIGY)<br>* En entier *<br>-- | 1,12,13 | C 07 D 401/04<br>C 07 D 471/04<br>C 07 D 401/14<br>C 07 D 409/14//<br>(C 07 D 471/04<br>C 07 D 249:00<br>C 07 D 221:00)<br>(C 07 D 471/04<br>C 07 D 235:00<br>C 07 D 221:00) |
| A | CHEMICAL ABSTRACTS, vol. 52, 1958, abrégé no. 2005f, Columbus, Ohio, US;<br>B.L. BASTIĆ et al.: "The condensation products of pyridinecarboxylic acids with 1,2-diaminonaphthalene", & GLASNIK KHEM. DRUSHTVA, BEOGRAD 18, NO. 4, 235-41, (1953)<br>* Compose IV, reg. no. 109602-03-1 *<br>-- | 1 | |
| A | EP-A-0 298 196 (ISHIHARA)<br>* Page 48, compose 115 *<br>-- ./. | 1 | DOMAINES TECHNIQUES RECHERCHES (Int. Cl.4)<br>C 07 D 401/00<br>C 07 D 471/00<br>C 07 D 409/00 |

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes: 13
Revendications ayant fait l'objet de recherches incomplètes: 1-12
Revendications n'ayant pas fait l'objet de recherches:
Raison pour la limitation de la recherche:

See sheet -C-

| Lieu de la recherche | Date d'achèvement de la recherche | Examinateur |
|---|---|---|
| LA HAYE | 07-01-1991 | DE JONG |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1505.1 03 82

Office européen des brevets

**RAPPORT PARTIEL DE RECHERCHE EUROPEENNE**

Numéro de la demande

EP 90 42 0452



| DOCUMENTS CONSIDERES COMME PERTINENTS | | | CLASSEMENT DE LA DEMANDE (Int. Cl.4) |
|---|---|---|---|
| Catégorie | Citation du document avec indication, en cas de besoin, des parties pertinentes | Revendication concernée | |
| X | CHEMICAL ABSTRACTS, vol. 52, 1958, abrégé no. 16349e, Columbus, Ohio, US;<br>B.L. BASTIĆ et al.: "Condensation of quinolinic acid anhydride with 3,4-toluenediamine and the hydrolysis of the resulting lactam", & GLASNIK KHEM. DRUSHTVA, BEOGRAD 21, 95-100(1956).<br><br>* 2-(methyl-2-benzimidazolyl) nicotinic acid, methyl ester, RN=111007-09-1; 2-(5-methyl-2-benzimidazolyl) nicotinanilide, RN=113091-77-3 * | 1 | DOMAINES TECHNIQUES RECHERCHES (Int. Cl.4) |
| | ---- | | |

<u>Raison</u> : La revendication 1 est tellement large qu'une recherche complète n'est guère possible. Les revendications 2-12 sont basées sur la revendication 1.
La recherche a été basée sur la structure I (décrite dans la première revendication) en tenant compte uniquement des variables Y et R1 telles que décrites dans les exemples.

- Revendications ayant fait l'objet de recherches complètes : 13
- Revendications ayant fait l'objet de recherches incomplètes : 1-12